# EUROPEAN PATENT APPLICATION

(11) **EP 2 514 454 A1**
(43) Date of publication of application: **24.10.2012**
(21) Application number: 11163398.8
(22) Date of filing: 21.04.2011
(51) Int. Cl.: A61M 5/24, A61M 5/34, A61M 5/00

(54) **Dedicated connector**

(71) Applicant: Sanofi-Aventis Deutschland GmbH, 65929 Frankfurt (DE)
(72) Inventor: Kouyoumjian, Garen, Warwickshire, CV31 1HN (GB); Boyd, Malcolm Stanley, Warwickshire, CV35 9PW (GB); De Sausmarez Lintell, Daniel Thomas, Warwickshire, CV23 9EQ (GB)

(57) **Abstract**

A dedicated connector may be provided to connect a dedicated needle assembly, such as a dedicated medicated module, to a cartridge holder. The dedicated connector may include a main body extending from a distal end to a proximal end, and the proximal end of the main body may be configured for attaching to a distal end of a drug delivery device. The dedicated connector may further include a dedicated mechanical coupling configured at the distal end of the main body, wherein the dedicated mechanical coupling is configured to form a connection to a proximal end of a dedicated needle assembly.

## Description

### Field of the Present Patent Application

One aspect of the present application relates to medical devices and methods of delivering at least two drug agents from separate reservoirs using devices having only a single dose setting mechanism and a single dispense interface. A single delivery procedure initiated by the user causes a non-user settable dose of a second drug agent and a variable set dose of a first drug agent to be delivered to the patient. The drug agents may be available in two or more reservoirs, containers, or packages, each containing independent (single drug compound) or pre-mixed (co-formulated multiple drug compounds) drug agents. One aspect of our present application is of particular benefit where the therapeutic response can be optimized for a specific target patient group, through control and definition of the therapeutic profile.

In another aspect of the present application, a medicated module may comprise a dedicated needle assembly. With such a dedicated needle assembly, the needle assembly may only be used to administer a dose of a medicament that is contained within an associated dedicated drug delivery device, such as a dedicated pen type drug delivery device. Alternatively, the needle assembly may comprise a non-medicated module wherein the needle assembly may only be used with a dedicated drug delivery device. In one preferred arrangement, the dedicated needle assembly comprises a double ended needle.

### Background

There are a number of potential problems that can arise when delivering two active medicaments or "agents" simultaneously. The two active agents may interact with each other during the long-term, shelf life storage of the formulation. Therefore, there are certain advantages to storing the active components separately and then combining them at the point of delivery, e.g. injection, need-less injection, pumps, or inhalation. However, the process for combining the two agents needs to be straightforward and convenient for the user to perform reliably, repeatedly and safely.

A further potential concern is that the quantities and/or proportions of each active agent making up the combination therapy may need to be varied for each user or at different stages of their therapy. For example, one or more active agents may require a titration period to gradually introduce a patient to a "maintenance" dose. A further example would be if one active agent requires a non-adjustable fixed dose while the other is varied in response to a patient's symptoms or physical condition. This potential problem means that pre-mixed formulations of multiple active agents may not be suitable as these pre-mixed formulations would have a fixed ratio of the active components, which could not be varied by the healthcare professional or user.

Additional concerns arise where a multi-drug compound therapy is required, because certain users cannot cope with having to use more than one drug delivery system or make the necessary accurate calculation of the required dose combination. This is especially true for users with dexterity or computational difficulties.

Other potential problems can arise where a user attempts to re-use a non-sterile needle assembly after a certain dose combination has been delivered. Using such non-sterile needle assemblies could lead to the transmission of certain diseases (septicemia) and therefore there exists a need for a medicated module that prevents needle re-use. There is a further concern of inadvertent needle sticks with certain needle assemblies where the injection needle is not concealed or covered, especially after use when a needle may be contaminated with blood. As such, there is also a general need to reduce certain patient's needle anxiety that may heighten a patient's fear or phobia of exposed needles. Applicants' proposed medicated modules help to reduce this anxiety.

As described herein, in one situation, a patient would attach a medicated module to the drug delivery device in order to deliver their required combination dose of medication comprising a selected dose of the first medicament and a fixed dose of the second medicament. Following the administration of this combined dose, the single dose of medication within the medicated module would have been used and so features on the medicated module (such as a locking needle guard and / or visual warnings) would help prevent the patient from being able to inject a second (non-combination) dose through the medicated module. A patient or user of the device would therefore be required to remove the used or spent medicated module and attach a new medicated module to the drug delivery device for each dose administration.

An increasing number of drug delivery devices, such as pen type drug delivery devices, are being marketed, including ones that are used for the delivery of different types of drugs. The issue of device and/or drug differentiation is becoming of increased importance as there arise certain safety issues (some life-threatening) associated with a patient or user mistaking one drug delivery device for another device and then administering an incorrect or wrong drug. While device/drug differentiation can be achieved in a number of ways, a preferable method of differentiation is mechanical prevention (i.e., making it difficult or nearly impossible for a device/drug mix up to occur). As just one example, a number of commercially available pen type drug delivery devices are supplied with a coupling mechanism that is non-proprietary, that is, the coupling mechanism accommodates the attachment of a conventional Type A needle assembly via a helical thread. For such 'mono-product' devices, the use of different Type A needle assemblies is acceptable, as the needle assembly in this instance is simply the means of administering the medicament from the primary reservoir of the drug delivery device.

This may not be the case for Applicants' presently disclosed medicated module and systems, where inadvertent use of a medicated module with a non-approved primary drug delivery device could have serious consequences (e.g., such consequences could include unknown health risks as the two formulations may not have been subject to any clinical evaluation or perhaps lacks regulatory approval). Equally, the use of a standard Type A needle with the approved primary drug delivery device may not be desirable, as a patient would not receive the targeted combination dose. In one situation, this might result in reduced therapeutic efficacy. However, in a worse situation, use of a standard Type A needle with the approved primary drug delivery device could result in non-desirable side effects (e.g., in the instance where the secondary medicament had some kind of balancing, cancelling or delaying effect on the pharmaco-kinetics ("PK") and/or pharmaco-dynamics ("PD") of the primary medicament contained within the drug delivery device). There are therefore certain safety and clinical benefits to configuring a combination delivery device so as to prevent attachment of the medicated module to an incorrect primary drug delivery device. There are also, therefore, certain benefits (e.g., regarding safety and clinical benefits) to configure a combination delivery device so as to prevent attachment of a standard or conventional Type A needle to the combination therapy's primary drug delivery device.

Accordingly, there exists a need to provide devices and methods for the delivery of two or more medicaments in a single injection or delivery step that is simple and safe for the user to perform and that also tends to reduce a patient's anxiety towards injections or needles or combinations of drug therapies. The presently disclosed dedicated needle assemblies and administration systems overcome the above-mentioned concerns by providing separate storage containers for two or more active drug agents that are then combined and/or administered during a single delivery procedure. Such devices may be provided in separate storage containers or provided in a kit form comprising at least one medicated module and at least one non-medicated module with dedicated attachments between each.

Setting a dose of one medicament automatically fixes or determines the dose of the second medicament (i.e., a non-user settable). The present application also gives the opportunity for varying the quantity of one or both medicaments. For example, one fluid quantity can be varied by changing the properties of the injection device (e.g., dialing a user variable dose or changing the device's "fixed" dose). The second fluid quantity can be changed by manufacturing a variety of secondary drug containing packages or kits with each variant containing a different volume and/or concentration of the second active agent. The user or healthcare professional would then select or prescribe the most appropriate secondary package or series or combination of series of different packages or kits for a particular treatment regime.

These and other advantages will become evident from the following more detailed description of the invention.

### SUMMARY

The present application discloses modules, systems, methods, drug delivery devices and kits that allow for the complex combination of multiple drug compounds within a single drug delivery system. Preferably, such a system includes a needle guard that functions to prevent needle assembly reuse and that can also function to reduce needle phobia while also reducing potential inadvertent needle sticks. Such a system may also include a containment of a (secondary) drug compound within a needle sub-assembly, what will be referenced as a medicated module in the context of this disclosure.

A user can set and dispense a multi-drug compound device through one single dose setting mechanism and a single drug dispense interface. Preferably, the single drug dispense interface may then be locked out so as to prevent reuse of a medicated module (i.e., re-use of the injection needle). This single dose setter controls the mechanism of the device such that a predefined combination of the individual drug compounds is delivered when a single dose of one of the medicaments is set and dispensed through the single drug dispense interface.

By defining the therapeutic relationship between the individual drug compounds the presently disclosed delivery devices and systems would help ensure that a patient/user receives the optimum therapeutic combination dose from a multi-drug compound device without the inherent risks associated with multiple inputs where the user has to calculate and set the correct dose combination when they use the device. The medicaments can be fluids, defined herein as liquids or gases that are capable of flowing and that change shape at a steady rate when acted upon by a force tending to change its shape. Alternatively, one of the medicaments may be a solid that is carried, solubilized or otherwise dispensed with another fluid medicament.

The present application is of particular benefit to patients with dexterity or computational difficulties as the single input and associated predefined therapeutic profile removes the need for them to calculate their prescribed dose when they use the device and the single input allows considerably easier setting and dispensing of the combined compounds. This application is also of particular benefit to patients experiencing needle phobia or who may experience a general fear of inadvertent needle sticks.

In a preferred embodiment a master or primary drug compound, such as insulin, contained within a multiple dose, user selectable drug delivery device could be used with a single use, user replaceable, medicated module that contains a single dose of a secondary medicament and the single dispense interface. When connected to the primary device the secondary compound is activated/delivered on dispense of the primary compound. Although the present application specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our invention.

For the purposes of our invention the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Pro-Ser-NH2), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Ser-Lys-Lys-Lys-Lys-Lys-Lys-NH2).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

In one arrangement, a dedicated needle assembly attachable only to a dedicated drug delivery device is disclosed and this dedicated needle assembly comprises a connecting body that extends from a distal end to a proximal end. A dedicated mechanical coupling is configured at the proximal end of the connecting body. This dedicated mechanical coupling is configured to form a releasable connection to a distal end of a dedicated drug delivery device. The dedicated needle assembly is pushed on the dedicated drug delivery device when the dedicated needle assembly is attached to the drug delivery device, and the dedicated needle assembly is pulled off the dedicated drug delivery device when the dedicated needle assembly is detached from the dedicated drug delivery device.

In an example, the dedicated mechanical coupling comprises a ring-shaped extension that is configured to form a releasable connection to an engaging ring-shaped cavity of the dedicated drug delivery device. In another example, the dedicated mechanical coupling comprises a ring-shaped extension that is configured to form a releasable connection to an engaging ring-shaped extension of the dedicated drug delivery device, wherein an inner edge of the ring-shaped extension comprises at least one recess, and wherein an outer edge of the engaging ring-shaped extension comprises at least one corresponding protrusion. In yet another example, the dedicated mechanical coupling comprises a ring-shaped extension that is configured to form a releasable connection to an engaging ring-shaped cavity of the dedicated drug delivery device, wherein the ring-shaped extension comprises a non-circular outer edge.

In another example, a dedicated connector may be provided to connect a dedicated needle assembly (e.g., such as a dedicated medicated module) to a drug delivery device. The dedicated connector may include a main body extending from a distal end to a proximal end, and the proximal end of the main body may be configured for attaching to a distal end of a drug delivery device. The dedicated connector may further include a dedicated mechanical coupling configured at the distal end of the main body, wherein the dedicated mechanical coupling is configured to form a connection to a proximal end of a dedicated needle assembly. Various dedicated connectors are possible. In an example, the dedicated connector may be configured to connect to a cartridge holder having a conventional mechanical coupling. In another example, the dedicated connector may be configured to connect to a cartridge holder having a modified mechanical coupling. Further, the dedicated connector may be permanently coupled to the cartridge holder; however, in other examples, the connector may be configured to be releasably connected to the cartridge holder. The permanent coupling may include mechanical coupling, such as screw or snap connection, bonding, or a combination of both. A snap connection may comprise at least one snap engagement feature and at least one corresponding snap aperture.

In one example, the dedicated connector may be permanently mounted on the conventional (i.e., standard Type-A) cartridge holder mount. For instance, the connector may be screwed onto the conventional screw thread mount and bonded into place. This connection and bonding may occur, for example, during an assembly process for the drug delivery device. In further examples the connector may comprise bonding surface or extensions, such as arms that enlarge the contact area between the connector and the distal end of the drug delivery device. Furthermore, these bonding surfaces may be configured to comprise latch features. However, alternative bonding surfaces could be threads on the proximal end of the connector that mate with corresponding threads on the distal end of the drug delivery device, e.g. a cartridge holder. Bonding could be for example adhesive, mechanical, and/or thermal bonding between threads or planar surfaces. Bonding may be applied during assembly. In one example bonding is used during assembly to ensure that connector can be (i) fitted to the standard type-A cartridge holder during assembly but not (ii) removed from that cartridge holder by the user.

In one example the connector acts as a "step-down" connector. This connector increases the operable length of the system in order to be able to fit the "step down" in diameter around a standard cartridge-holder mechanical coupling. In this example, this means that the proximal needle on the dedicated needle assembly may need to be longer in order to sufficiently penetrate the septum on the primary cartridge in the drug delivery device. Thus, a conventional Type-A injection needle would not be allowed to penetrate the septum on a primary cartridge in the drug delivery device through the connector and the cartridge holder. Alternatively, the proximal needle may be proved on the connector, for example on a connector hub. In other examples, the dedicated connector could be a "step-up" connector. That is, the diameter of the dedicated mechanical coupling of the dedicated connector may be greater than the diameter of the mechanical coupling of the drug delivery device. However, this "step up" connector also increases the operable length of the system, thus preventing a conventional Type-A injection needle to penetrate the septum on a primary cartridge in the drug delivery device through the connector and the cartridge holder.

In one example the connector may comprise a frangible element, e.g. the neck portion of the main body of the connector may comprise at least one frangible element. In one further example the connector may comprise at least one engagement feature and at least one frangible element. The frangible element is cofigured to remain attached to the drug delivery device once the connector was attached and is then removed.

In one example the dedicated connector is configured to prevent a needle of a conventional needle assembly, e.g. Type-A connector injection needle, from piercing a cartridge septum of a cartridge disposed in the drug delivery device, e.g. a pen-type injection device.

A particular benefit of our application is that the medicated module makes it possible to tailor dose regimes when required, especially where a titration period is necessary for a particular drug. The medicated module could be supplied in a number of titration levels with differentiation features such as, but not limited to, aesthetic design of features or graphics, numbering etc, so that a patient could be instructed to use the supplied medicated module in a specific order so as to facilitate titration. Alternatively, the prescribing physician may provide the patient with a number of "level one" titration medicated modules or a kit of modules and then when these were finished, the physician could then prescribe the next level or the next drug delivery kit. One advantage of this titration program is that the primary device can remain constant throughout.

Another particular benefit of Applicants' proposed dedicated needle assemblies and assembly systems is that they provide a mechanical device-based solution that can be used to achieve certain advantages. Such advantages include facilitating attachment of the dedicated needle assembly (e.g., a medicated module) to a correct primary drug delivery device by a patient or user. The presently disclosed dedicated needle assemblies also help prevent accidental or inadvertent attachment of the dedicated needle assembly to non-approved drug delivery device that comprises a conventional needle assembly fitting, such as a standard or conventional Type A fitting. In addition, dedicated needle attachment features also prevent a patient or user from accidentally attaching a standard Type A needle to the combination therapy's primary drug delivery device.

In order to facilitate split dosing scenarios (e.g., end of cartridge scenario, split for volume, etc), specific non-medicated modules or "zero-dose" needle assemblies could be supplied and/or made available to patients for use with the combination delivery system which utilize the same or similar dedicated mechanical attachment features. The term non-medicated module is used for a needle sub-assembly that does not comprise a containment of a (secondary) drug compound. Supply of such non-medicated needle assemblies might either be in a controlled manner, e.g. supply of a single 'zero-dose' needle alongside each replacement primary drug delivery device (to accommodate end of cartridge dose splitting, if required), or in a managed manner, e.g. via prescription (e.g., for patients whose regular dose of the medication in the primary drug device is greater than the maximum dose the device can deliver in a single injection and who therefore are forced to split their dose into 2 or more separate injections), or in a direct access manner (e.g., over the counter from a Pharmacy). In addition, where 'zero-dose' needles are dedicated and also single use, this tends to help control the ability to use them indiscriminately.

Additionally, if the dedicated needle assemblies were to be used for scenarios where multiple secondary medicaments could be used (e.g., a long acting insulin along with a first drug type "Drug A", a long acting insulin along with a second drug type "Drug B", a long acting insulin along with a third drug type 'Drug C'), or where multiple (but independently exclusive) combination therapies were to be marketed ('Drug A' plus 'Drug X', 'Drug B' plus 'Drug Y', 'Drug C' plus 'Drug Z', etc.) then it might be desirable for the exclusive or proprietary dedicated mechanical coupling to also include specific coding features and/or mechanical attributes to maintain exclusivity while also allowing a level of controlled differentiation within a family of combination therapies. One advantage of such a situation is that this would potentially enable the same basic dedicated mechanical coupling of the dedicated needle assemblies to be used (e.g., push to fit, pull to detach) across all supplied drug delivery devices or all devices contained within a family of drug delivery devices. This would thereby help preserve the usability benefits of the selected approach, while also providing a means for mechanical differentiation and/or dedication to help reduce the risk of patient mix up between individual drugs from the family of combination therapies that used the system.

In a preferred embodiment, the primary drug delivery device is used more than once and therefore is multi-use. Such a device may or may not have a replaceable reservoir of the primary drug compound, but our invention is equally applicable to both scenarios. It is possible to have a suite of different medicated modules for various conditions that could be prescribed as one-off extra medication to patients already using a standard drug delivery device (or family of devices). Should the patient attempt to reuse a previously used medicated module, the presently disclosed medicament module can provide a lockable needle guard feature that could alert the patient to this situation. Other means of alerting the user may include some (or all) of the following:
1. Physical prevention of medicated module re-attachment to the primary drug deliver device once the module was used and removed.
2. Physical prevention of insertion of the used drug dispense interface into the patient (e.g., a single use needle-guard type arrangement).
3. Physical / hydraulic prevention of subsequent liquid flow through the drug dispense interface once it has been used.
4. Physical locking of the dose setter and/or dose button of the primary drug delivery device.
5. Visual warnings (e.g., change in color and/or warning text/indicia within an indication window on the module once needle insertion and/or fluid flow has occurred).
6. Tactile feedback (presence or absence of tactile features on the outer surface of the module hub following use).

These as well as other advantages of various aspects of the present invention will become apparent to those of ordinary skill in the art by reading the following detailed description, with appropriate reference to the accompanying drawings.
The present invention provides for easy and safe handling of drug delivery devices, e.g. for self-adminsitration. The invention gives manufacturing flexibility that allows to combine standard products, e.g. a pen-type drug delivery device, and a non-standard medicated module via an interface connector component that provides the dedicated mechanical coupling required to connect both products.

### BRIEF DESCRIPTION OF THE DRAWINGS

Exemplary embodiments are described herein with reference to the drawings, in which:
Figure 1 illustrates a sectional view of one needle assembly arrangement comprising a medicated module that is attached to a drug delivery device having a conventional screw thread;
Figure 2 illustrates a perspective view of the medicated module of Figure 1 having two needles connected to a reservoir attached to a drug delivery device;
Figure 3 illustrates a front view of the medicated module of Figure 2;
Figure 4 illustrates the medicated module illustrated in Figure 1 having a locked out needle guard;
Figure 5 illustrates a non-medicated module that may be provided in a drug delivery kit that includes the medicated module illustrated in Figure 1;
Figure 6 illustrates a partial view of a movable lockout member of the non-medicated module illustrated in Figure 5;
Figure 7 illustrates a perspective view of the movable lockout member illustrated in Figure 6;
Figure 8 illustrates a front view of the module illustrated in Figure 5 having a locked needle guard;
Figure 9 illustrates one possible drug delivery device that can be used with the medicated module illustrated in Figure 1;
Figure 10 illustrates a perspective view of one arrangement of a dedicated needle assembly configured to be attachable to a dedicated drug delivery device;
Figure 11 illustrates a perspective view of a distal end of one arrangement of a dedicated drug delivery device that is configured to be attachable to the dedicated needle assembly illustrated Figure 10;
Figure 12 illustrates a perspective view of the dedicated drug delivery device illustrated in Figure 11 just prior to being inserted into the dedicated needle assembly illustrated in Figure 10 where the dedicated drug delivery device is not aligned with the dedicated needle assembly;
Figure 13 illustrates a perspective view of the dedicated drug delivery device partially inserted into the dedicated needle assembly illustrated in Figure 10 where the dedicated drug delivery device is still not aligned with the dedicated needle assembly;
Figure 14 illustrates a perspective view of the dedicated drug delivery device after the device has been rotated to align its dedicated mechanical coupling with that of the dedicated needle assembly illustrated Figure 10;
Figure 15 illustrates close up view of the dedicated drug delivery device after the device has been rotated to align its dedicated mechanical coupling with that of the dedicated needle assembly illustrated Figure 10;
Figure 16 illustrates a perspective view of the distal end of the dedicated drug delivery device after the device has been fully inserted into the dedicated needle assembly illustrated in Figure 10;
Figure 17 illustrates a perspective view of one arrangement of a dedicated cap that may be used to connect a dedicated drug delivery device to the dedicated needle assembly illustrated Figure 10;
Figure 18 illustrates a cut away view of the dedicated cap illustrated in Figure 17;
Figure 19 illustrates a partial sectional view of the dedicated cap illustrated in Figure 17;
Figure 20 illustrates a perspective view of a standard needle arrangement that can be used with a drug delivery device having a conventional threaded distal end, such as the device illustrated in Figure 9;
Figure 21 illustrates a perspective view of one arrangement of a dedicated needle assembly configured to be attachable to a dedicated drug delivery device;
Figure 22 illustrates a perspective view of a distal end of one arrangement of a dedicated drug delivery device that is configured to be attachable to the dedicated needle assembly illustrated Figure 21;
Figure 23 illustrates a perspective view of the dedicated drug delivery device illustrated in Figure 22 just prior to being inserted into the dedicated needle assembly illustrated in Figure 21;
Figure 24 illustrates a perspective view of one arrangement of a dedicated needle assembly configured to be attachable to a dedicated drug delivery device;
Figure 25 illustrates a perspective view of a distal end of one arrangement of a dedicated drug delivery device that is configured to be attachable to the dedicated needle assembly illustrated Figure 24;
Figure 26 illustrates a perspective view of the dedicated drug delivery device illustrated in Figure 25 just prior to being inserted into the dedicated needle assembly illustrated in Figure 24;
Figure 27 illustrates a perspective view of one arrangement of a dedicated needle assembly configured to be attachable to a dedicated drug delivery device;
Figure 28 illustrates a perspective view of a distal end of one arrangement of a dedicated drug delivery device that is configured to be attachable to the dedicated needle assembly illustrated Figure 27;
Figure 29 illustrates a perspective view of the dedicated drug delivery device illustrated in Figure 28 just prior to being inserted into the dedicated needle assembly illustrated in Figure 27;
Figure 30 illustrates a perspective view of one arrangement of a dedicated connector for a dedicated needle assembly and a drug delivery device;
Figure 31 illustrates a perspective view of the proximal end of the dedicated connector of Figure 30 and the distal end of the drug delivery device of Figure 30;
Figure 32 illustrates a perspective view of another arrangement of a dedicated connector for a dedicated needle assembly and a drug delivery device;
Figure 33 illustrates the dedicated connector of Figure 32 connected to the drug delivery device of Figure 32;
Figure 34 illustrates a perspective view of the dedicated connector of Figure 32 and the distal end of the drug delivery device of Figure 32;
Figure 35 illustrates a cross-sectional view of the dedicated connector of Figure 32, where the dedicated connector connects the dedicated needle assembly and the drug delivery device;
Figure 36 illustrates a cross-sectional view of the dedicated connector of Figure 32 and a standard type-A needle assembly;
Figure 37 illustrates a perspective view of another arrangement of a dedicated connector for a dedicated needle assembly and a drug delivery device;
Figure 38 illustrates a cross-sectional view of the dedicated connector of Figure 37, where the dedicated connector connects the dedicated needle assembly and the drug delivery device;
Figure 39 illustrates a perspective view of another arrangement of a dedicated connector for a dedicated needle assembly and a drug delivery device;
Figure 40 illustrates a cross-sectional view of the dedicated connector of Figure 39, where the dedicated connector connects the dedicated needle assembly and the drug delivery device;
Figure 41 illustrates a perspective view of another arrangement of a dedicated connector for a dedicated needle assembly and a drug delivery device;
Figure 42 illustrates a cross-sectional view of the dedicated connector of Figure 41, where the dedicated connector connects the dedicated needle assembly and the drug delivery device;
Figure 43 illustrates a perspective view of the dedicated connector of Figure 41 attached to the drug delivery device of Figure 41;
Figure 44 illustrates a perspective view of the dedicated connector of Figure 41 detached from the drug delivery device of Figure 41;
Figure 45 illustrates a cross-sectional view of the dedicated connector of Figure 41 detached from the drug delivery device of Figure 41;
Figure 46 illustrates a cross-sectional view of a standard type-A needle assembly attached to the drug delivery device of Figure 45;
Figure 47 illustrates a cross-sectional view of the drug delivery device of Figure 45 attempting to connect to another dedicated connector;
Figure 48 illustrates a perspective view of another arrangement of a dedicated connector for a dedicated needle assembly and a drug delivery device;
Figure 49 illustrates a cross-sectional view of the dedicated connector of Figure 48, where the dedicated connector connects the dedicated needle assembly and the drug delivery device;
Figure 50 illustrates a cross-sectional view of the dedicated needle assembly of Figure 48 and the drug delivery device 48 attached to one another without a dedicated connector; and
Figure 51 illustrates a cross-sectional view of the dedicated needle assembly of Figure 48 and the drug delivery device 48 and a conventional needle assembly.

### DETAILED DESCRIPTION

The present invention administers a fixed predetermined dose of a second medicament (secondary drug compound) and a potentially variable dose of a first medicament (primary drug compound) through a single output or drug dispense interface such as a double ended needle. Setting the dose of the primary medicament by the user automatically determines the fixed dose of the second medicament. This fixed dose of the second medicament is preferably a single dose. In a preferred arrangement, the drug dispense interface comprises a needle cannula (hollow needle) and a needle guard that may be locked out after medicament injection.

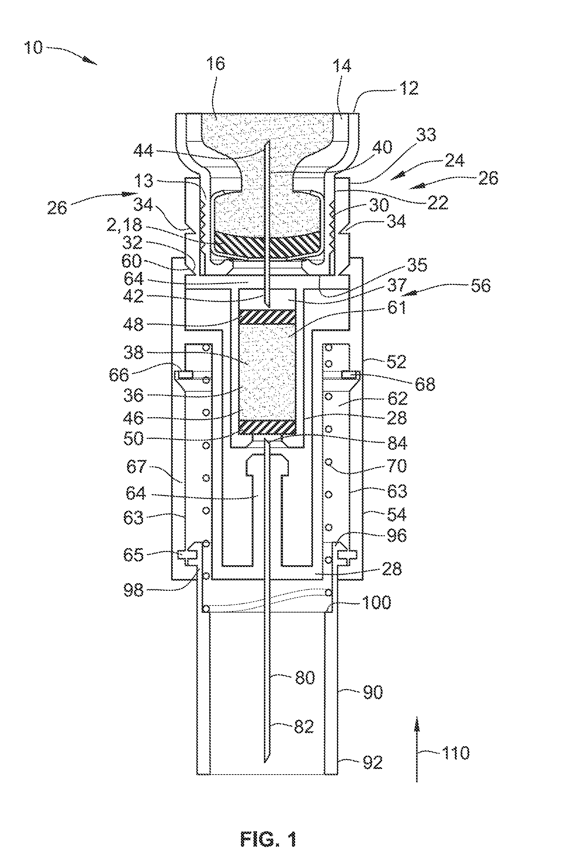

Figure 1 illustrates a sectional view of one needle assembly arrangement comprising a medicated module 10 that is attached to a drug delivery device 12 having a conventional screw thread 30. As illustrated, the medicated module 10 comprises a first needle 40 that pierces a septum 2 of a device cartridge 14. A second injection needle 80 is used to subcutaneously inject the first medicament contained in the cartridge along with a second medicament contained in the medicated module. Located between the two needles 40, 80 is a recess 37 defined by a connection body 24. Preferably, this recess contains a reservoir of a second medicament 38. Most preferably, this reservoir comprises a capsule 46 that has ends sealed with first and a second pierceable seals 48, 50, respectively.

In this preferred arrangement, the medicated module 10 as illustrated is attached to a drug delivery device 12. Only a portion of such a drug delivery device is illustrated in Figure 1. The drug delivery device 12 comprises a cartridge holder containing a standard cartridge 14. This standard cartridge 14 comprises a first medicament 16 such as insulin or the like.

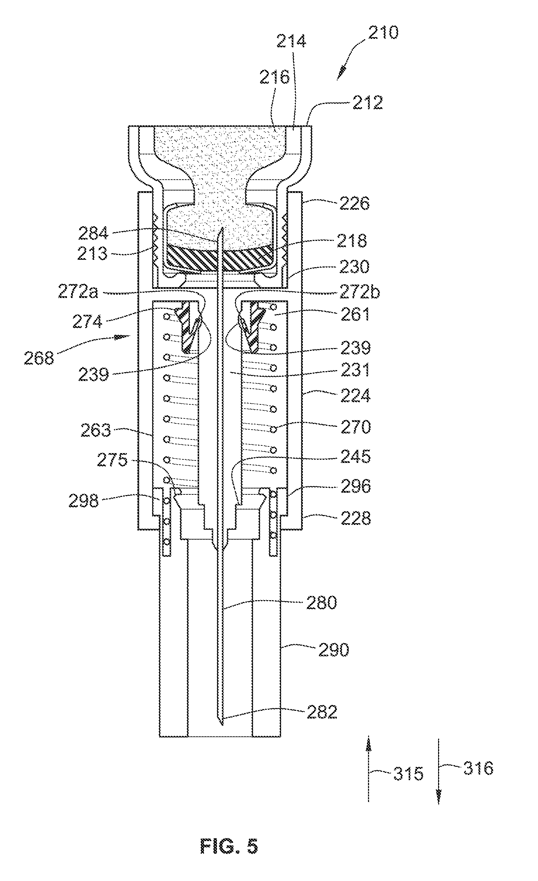

In one arrangement, the medicated module 10 is preferably self-contained and may be provided as a sealed and sterile disposable module. Such a module comprises an attachment means compatible to the attachment means at a distal end of the drug deliver device 12. As described in greater detail below, the medicated module 10 could be supplied by a manufacturer contained in a protective and sterile capsule or container where the user would peel or rip open a seal or the container itself to gain access to the sterile medicated module. In some instances it might be desirable to provide two or more seals for each end of the medicated module. In addition, and as will be explained in greater detail below, in one arrangement, such medicated module 10 may be provided in a drug delivery kit along with at least one non-medicated module, such as the non-medicated module illustrated in Figure 5.

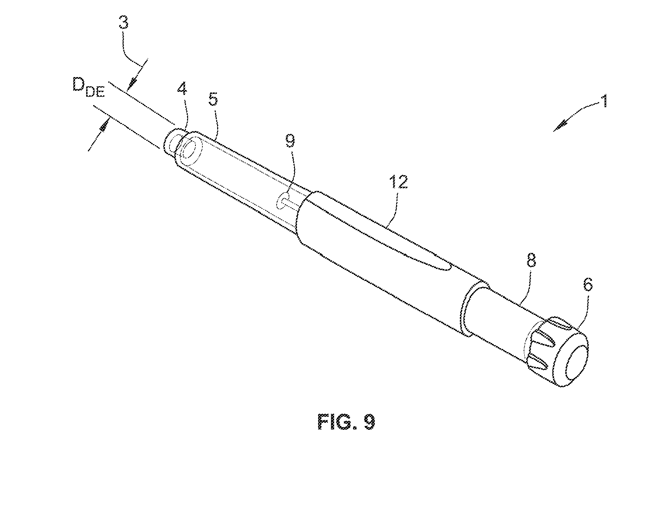

One example of a drug delivery device 1 is illustrated in Figure 9. Referring to Figure 9, there is shown a conventional drug delivery device 1 in the form of a pen type drug delivery device. This drug delivery device 1 comprises a dose setting mechanism 6, a cartridge holder 5, and a removable cap 3. The cartridge holder comprises a tubular member 7 that extends from a proximal end to a distal end. The distal end of the cartridge holder 5 comprises a coupling mechanism 4 for releasably coupling a dispense interface, such as a double ended needle assembly. In this conventional drug delivery device 1, this distal end 4 defines a distal end diameter DDE 10 and this coupling mechanism comprises a conventional screw thread.

The proximal end of the cartridge holder and the distal end of the dose setting mechanism 12 are secured together. The pen type drug delivery device may comprise a re-usable or a disposable pen type drug delivery device. Where the drug delivery device comprises a re-usable device, the cartridge holder 5 and the dose setting mechanism 12 are releasably coupled together. In a disposable device, they are permanently coupled together. The dose setting mechanism comprises an outer housing that extends from a proximal end to a distal end of the dose setting mechanism 12. In one preferred arrangement, the housing contains a spindle 9, such as a threaded spindle that rotates when a dose is injected.

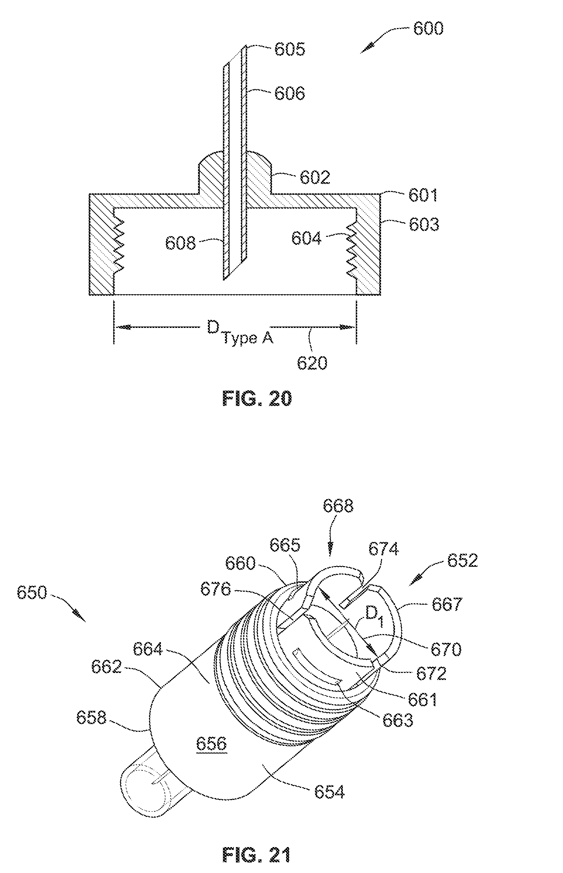

To inject a previously set dose, a conventional double ended needle assembly is attached to the conventional thread screw 4 provided at the distal end of the tubular member of the cartridge holder. Figure 20 illustrates such a conventional needle assembly 600.

Figure 20 illustrates a cross sectional view of a conventional double ended needle assembly 600. The needle assembly 600 illustrated in Figure 20 comprises a double ended needle 606 and a hub 601. The double ended needle or cannula 606 is fixedly mounted in a needle hub 601. This needle hub 601 comprises a circular disk shaped element which has along its periphery a circumferential depending sleeve 603. Along an inner wall of this hub member 601, a conventional thread 604 is provided. This thread 604 allows the needle hub 601 to be screwed onto the distal end of the drug deliver device which is provided with a corresponding outer thread along a distal hub. At a center portion of the hub element 601 there is provided a protrusion 602. This protrusion 602 projects from the hub in an opposite direction of the sleeve member. A double ended needle 606 is mounted centrally through the protrusion 602 and the needle hub 601. This double ended needle 606 is mounted such that a first or distal piercing end 605 of the double ended needle forms an injecting part for piercing an injection site (e.g., the skin of a user).

Similarly, a second or proximal piercing end 608 of the needle assembly 600 protrudes from an opposite side of the circular disc so that it is concentrically surrounded by the sleeve 603. In one needle assembly arrangement, the second or proximal piercing end 606 may be shorter than the sleeve 603 so that this sleeve to some extent protects the pointed end of the back sleeve.

Returning to Figure 1, the illustrated arrangement has the benefit of the second medicament 38 as a single dose being contained entirely within the medicated module 10. This can minimize the risk of material incompatibility between the second medicament and the materials used in the construction of the medicated module 10. The medicated module 10 comprises a connecting body 24, a first needle 40, an outer body 52, a second needle 80, a biasing member 70, and a needle guard 90.

The connecting body 24 extends from a proximal end 26 to a distal end 28 added. The proximal end of the connecting body is provided with a connector 30 so that the medicated module 10 may be connected to the drug delivery device 12. Preferably, this connector is provided along an inner surface 22 of the connecting body 24 and provides a releasable connection to the drug delivery device 12. Such a releasable connector may comprise a snap fit, form fit, snap lock, luer lock or other similar connection mechanism known to those of skill in the art. As can also be seen from Figure 1, the connecting body 24 further comprises a first and second recess 32, 34. These recesses are provided along a connector body external surface 33. Although only two recesses 32, 34 are illustrated in the medicated module 10 arrangement illustrated in Figure 1, alternative recess arrangements may comprise more or less than two recesses 32, 34. As will be explained in greater detail below, as illustrated in Figure 1, a male member 60 of an outer body 52 is releasably engaged to the first recess 32.

The connecting body 24 defines a reservoir 36 and preferably this reservoir contains a second medicament 38. Most preferably, this second medicament 38 comprises a single dose of a medicament, such as a single dose of GLP-1 or alternatively a pre-mix of medicaments. In one preferred arrangement, the reservoir comprises a capsule 46 comprising a first and a second end that is sealed with pierceable membranes 48, 50. Such a construction provides a hermetically sealed reservoir for the second medicament 38.

The connecting body 24 further comprises a first needle 40 rigidly affixed in an upper surface 35 of the connecting body. Preferably, this first needle 40 comprises a double ended needle having a first piercing end 42 (i.e., a distal end) and a second piercing end 44 (i.e., a proximal end). In this preferred arrangement, when the medicated module 10 is initially mounted to the drug delivery device 12 as illustrated in Figure 1, the first piercing end 42 pierces the membrane 18 of the cartridge 14 but the second piercing end 44 does not yet pierce the first or proximal seal 48 of the capsule 46. As such, the first medicament 16 of the cartridge 14 is not in fluid communication with the second medicament 38 contained in the capsule 46.

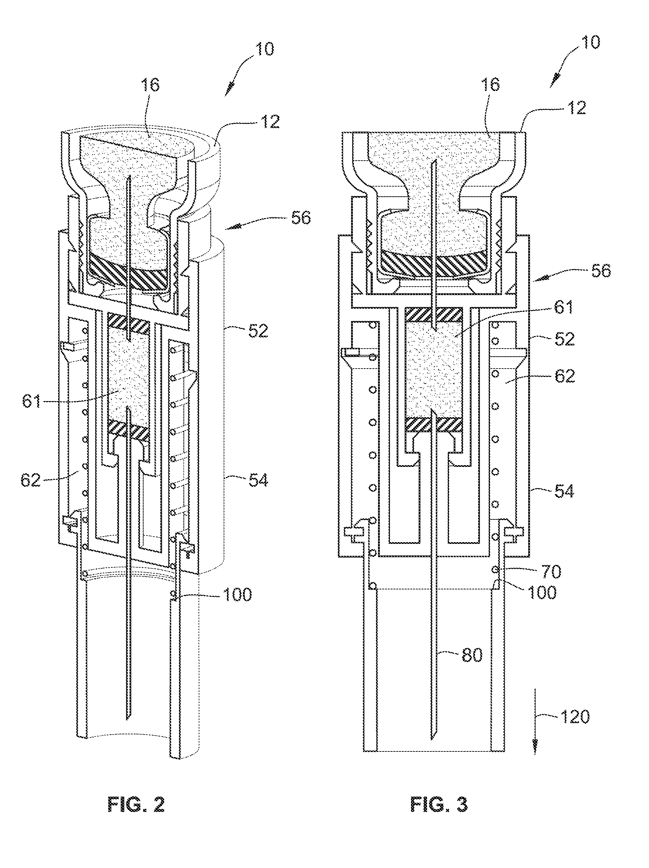

The medicated module 10 further comprises an outer body 52 and preferably this outer body is slidably engaged with the connecting body 24. More preferably, this outer body 52 is slidably engaged with the connecting body 24 and is slidable from an initial position (as illustrated in Figure 1) to a second or dose injecting position (as illustrated in Figures 2 and 3).

The outer body 52 comprises a distal end 54 and a proximal end 56. The outer body proximal end 56 is configured with a male member 60 that releasably engages the connecting body 24. Preferably, when the medicated module is initially mounted onto the drug delivery device 12 as illustrated in Figure 1, the male member 60 releasably engages the first recess 32 provided along the outer surface 33 of the connecting body 24. In the second or dose injecting position (as illustrated in Figures 2 and 3), the male member 60 is moved proximally so that this member engages the second recess 34.

The outer body 52 further comprises a first and a second inner cavity 61, 62 (added) respectively. Preferably, the first inner cavity 61 is formed to contain the reservoir of the connecting body 24 whereas the second inner cavity 62 is formed to contain an elastic member 70, such as a compression spring. As illustrated in Figure 1, in the initial mounted position of the medicated module, the elastic member 70 is in an extended state. In this extended state, the elastic member resides within this second cavity 62 and between the outer body 52 and the needle guard 90.

The outer body 52 further comprises a distal and a proximal groove 65, 66 provided on inner surface 52. The proximal groove 65 includes a movable locking mechanism 68, preferably in the form of a movable circlip. As will be explained below, this movable locking mechanism 68 is used to lock out the needle guard 90 after injection: that is, after the needle guard is first moved in a proximal direction and then returned in a distal direction.

The outer body 60 further comprises a second or injection needle 80 rigidly affixed in outer body hub element 64. Preferably, this second needle 80 comprises a double ended needle having a first piercing end 82 (i.e., a distal end) and a second piercing end 84 (i.e., a proximal end).

In this preferred arrangement, when the medicated module 10 is initially mounted to the drug delivery device 12 as illustrated in Figure 1, the second piercing end 84 does not yet pierce the distal seal 48 of the capsule 46. In addition, in this preferred arrangement, the first piercing end 82 of the second needle 80 is illustrated as being substantially concealed from a user's view by way of the needle guard 90 so as to help reduce any needle anxiety that a patient may be experiencing.

Preferably, needle guard 90 comprises a tubular shaped element and in a relaxed position, as illustrated in Figure 1, substantially conceals the second needle 80. While substantially concealing the second needle, the needle guard also helps to prevent inadvertent needle sticks. In Figure 1, this needle guard 90 is illustrated in an unlocked position. That is, during an injection step where a user initiates the injection, the needle guard 90 may be free to be moved in a proximal direction or towards the drug delivery device (illustrated by arrow 110 in Figure 1).

Preferably, the needle guard 90 comprises a plurality of outwardly directed arms 96, 98 (added). These arms 96, 98 are in sliding engagement with an inner surface 63 of the inner cavity 62 of the outer body 52 and reside within the second cavity 62 defined by the outer body. These outwardly directed arms 96, 98 allow for the needle guard 90 to be placed and held in a locked out position after dose injection. In addition, these outwardly directed arms 96, 98 may also serve as a rotation preventor so as to prevent the needle guard from rotating either when it is connected to the drug delivery device or during the medicament injection step.

As shown in Figure 1, in this first position, the outer body 52 comprises at a proximal end inwardly extending male members 32 that engage the first recess 32 provided near the proximal end of the connecting body. When the outer body 24 resides in this initial connected position, the inwardly extending male members 32 engage the first set of recesses 32 of the connecting body and both the first and the second needles 40, 80 are not in fluid communication with the medicated module reservoir 36.

As discussed above, in the initial mounting position, both the first and the second needles 40, 80 are not in fluid communication with the medicated module reservoir 36. Figure 3 illustrates a side view of attachment of the medicated module 10 to the drug delivery device 12 in a dose ready state. To achieve this dose ready state or second state, the outer body 52 is moved in the proximal direction. This outer body proximal movement causes the inwardly extending male members 60 of the outer body to move from the first recess 32 to the second recess 34 of the connecting body.

Importantly, proximal movement of the outer body also causes the distal end 42 of the first needle 40 to penetrate the first pierceable seal 48 of the capsule 46 while the proximal end 42 of first needle 40 maintains its penetration of the septum of the cartridge 14 of the device 12. Proximal movement of the outer body also causes the proximal end 84 of the second needle 80 to penetrate the second pierceable seal 50 of the capsule 46. Piercing of membranes 48 and 50 opens fluid communication between the first and second medicaments 16, 38 allowing these two medicaments to be dispensed through operation of the dispense mechanism on the drug delivery device 12.

Where the drug delivery device 12 comprises a dose setter 8, a dose of the drug delivery device 1 may then be set using a dose setter 8 (see Figure 9) in the normal manner (e.g., by dialing out the appropriate number of units). Dispense of the medicaments 16, 38 may then be achieved by subcutaneously injecting the medicaments via activation of a dose button on device 12. The dose button 6 may be any triggering mechanism that causes the dose of the first medicament that was set by the dose setter to move distally towards the distal end of the device. In a preferred embodiment, the dose button is operably connected to a spindle that engages a piston in the primary reservoir of the first medicament.

The medicated module and the non-medicated module described herein should be designed to operate in conjunction with a multiple use injection device or family of devices, preferably a pen-type multi-dose injection device, similar to what is illustrated in Figure 9. The injection device could be a reusable or disposable device. By disposable device it is meant an injection device that is obtained from the manufacturer preloaded with medicament and cannot be reloaded with new medicament after the initial medicament is exhausted. The device may be a fixed dose or a settable dose, but in either case it is a multi-dose device.

A typical injection device contains a cartridge or other reservoir of medication. This cartridge is typically cylindrical in shape and is usually manufactured in glass. The cartridge is sealed at one end with a rubber bung and at the other end by a rubber septum. The injection pen is designed to deliver multiple injections. The delivery mechanism is typically powered by a manual action of the user, however, the injection mechanism may also be powered by other means such as a spring, compressed gas or electrical energy.

During injection, the needle guard 90 is moved in a proximal direction 110 against a force created by the elastic member 70. As the needle guard moves proximally, its outwardly directed arms 96, 98 slide internally within the second cavity 62 of the outer body 52 from the distal groove 65 to the proximal groove 66. Once the outwardly directed arms 96 reach the proximal groove 66, the outwardly directed arms 96 pick up the movable locking feature 68. The first and second medicament 16, 38 may then be injected into an injection site by way of the second needle 80.

After injection and the drug delivery device and the medicated module are removed from the injection site, the needle guard 90 under the force of the biasing element 70 is forced in the distal direction 120. On being forced down or in the distal direction (represented by arrow 120 in Figure 3) by the force created by the element 70, the needle guard 90 pulls the movable lockout member 68 into the distal groove 65 to thereby lock the needle guard 90 in the down position.

Locking the needle guard 90 in the down position in this manner provides a number of beneficial features. First, it prevents a user from re-using a non-sterile medicated module. Second, the locked needle guard protects and substantially conceals the second needle 80 and therefore reduces the risk of a potential inadvertent needle stick. And third, in substantially concealing the second needle 80, the locked needle guard acts to reduce any potential needle fear, needle phobia or needle anxiety that a patient may experience.

In the arrangements described herein, the second medicament may be either in a powdered solid state, any fluid state contained within the secondary reservoir or microcapsule, or coated to the inside surface of the drug dispense interface. The greater concentration of the solid form of the medicament has the benefit of occupying a smaller volume than the liquid having lower concentration. This in turn reduces the ullage of the medicated module. An additional benefit is that the solid form of the second medicament is potentially more straightforward to seal in the secondary reservoir than a liquid form of the medicament. The device would be used in the same manner as the preferred embodiment with the second medicament being dissolved by the first medicament during dispense.

The shape of the medicated module may be a cylindrical body or any other geometric shape suitable for defining a fluid reservoir or for containing discrete self-contained reservoir of the secondary medicament and for attaching one or more needle cannula. The reservoir in the medicated module can be manufactured from glass or other drug contact suitable material. The integrated injection needle can be any needle cannula suitable for subcutaneous or intramuscular injection.

Preferably the medicated module is provided by a manufacturer as a stand-alone and separate device that is sealed to preserve sterility. The sterile seal of the module is preferably designed to be opened automatically, e.g. by cutting, tearing or peeling, when the medicated module is advanced or attached to the drug delivery device by the user. This opening of the seal may be assisted by features such as angled surfaces on the end of the injection device or features inside the module.

Alternatively, the medicated module may be provided in a kit form along where such a kit comprises at least one non-medicated module or a safety needle assembly. There are a number of reasons to provide one or more non-medicated needle assemblies along with a medicated module (such as illustrated in Figure 1) in a kit form.

For example, there may be a situation where a patient may need to split a dose or top up a dose between two or more drug delivery devices. For example, there may be a situation where a user may need to administer a dose greater than the medicament remaining in the cartridge of the drug delivery device. As just one example, consider that a user might face a situation where they may need to administer a 50 Unit dose and only have only 30 Units remaining in the cartridge of their old (i.e. part-used) drug delivery device. In such a situation, the user would first mount the medicated module onto the drug delivery, set the drug delivery device to administer 30 Units of the first medicament and then administer the first and the second medicament in a generally known way. Then, because the user would still need to deliver the remaining 20 Units of the first medicament, rather than use another medicated module containing a dose of the second medicament, the user would simply mount a non-medicated module to a new drug delivery device and then administer the remaining 20 Units of the first medicament.

A user may also be faced with administering a large dose of the first medicament and may, for one reason or another, want to split this large dose (i.e., a large volume of medicament) into two or more injections. For example, some users may face themselves administering large doses on the order of 100 Units or more of a single medicament for a single injection. Rather than administer such a large volume of medicament during a single injection, the user may first administer 60 Units first while using the medicated module and then administer the remaining 40 Units using a non-medicated module. Splitting up the volume of the administered dose helps to reduce patient discomfort and may reduce potential medicament pooling under the skin. Splitting such a large dose may also be required where there is a mechanical restraint on the drug delivery device in that the device may not be mechanically capable of setting and administering such a large volume of medication.

Another reason that a user may need to split a dose between a medicated and a non-medicated needle is that perhaps a physician has instructed a user to split a dose up into two or more injections. Two or more injections may be required if a user experiences certain negative reactions when administering a full dose of a first medicament simultaneously with a second medicated dose. Alternatively, the patient may be instructed to initially administer a first medicament during a specific time of day (e.g., a long acting insulin in the morning) and then later in the day instructed to administer a combination of a first and second medicament (e.g., a long acting insulin in combination with a short acting insulin later in the day). In such a scenario, the non-medicated module could be used to administer the first injection.

Figure 5 illustrates a first arrangement of a non-medicated module and is somewhat similar in construction to the medicated module. For example, this module 210 comprises a connecting body 224, a double ended needle 280, a biasing member 270, a movable locking member 268, and a needle guard 290.

The connecting body 224 of the module 210 extends from a proximal end 226 to a distal end 228. The proximal end of the connecting body is provided with a connector 230 so that the connector body may be connected to the drug delivery device 212. Preferably, this connector 230 is provided along an inner surface 222 of the connecting body 224 and provides a releasable connection to the drug delivery device 212. Such a releasable connector may comprise a snap fit, form fit, snap lock, luer lock or other similar connection mechanism known to those of skill in the art.

The connecting body 224 further comprises an injection needle 280 rigidly affixed within a main stem 231 of a needle hub 235. Preferably, this needle 280 comprises a double ended needle having a first piercing end 282 (i.e., a distal end) and a second piercing end 284 (i.e., a proximal end). In this preferred arrangement, when the module 210 is initially mounted to the drug delivery device 212 as illustrated in Figure 5, the second piercing end 244 pierces the membrane 218 of the cartridge 214.

The connection body 224 further comprises a first inner cavity 261. Preferably, the first inner cavity 261 is formed to contain a movable locking element 268 and a biasing member 270, such as a compression spring. As illustrated in Figure 5, in the initial mounted position of the needle assembly, the biasing member 270 is in an extended state.

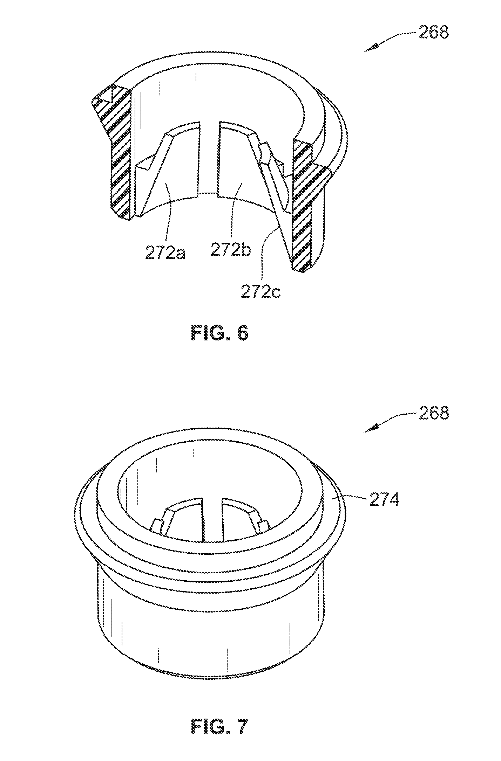

Details of a preferred arrangement of a locking mechanism can be clearly seen from Figures 6 and 7. Figure 6 illustrates a cross sectional view of the movable locking mechanism 268 and Figure 7 illustrates a perspective view of the locking mechanism 268. As illustrated, the movable locking mechanism 268 is preferably in the form of a cylindrical shaped member having an outer beveled edge 274. Preferably, the locking mechanism 268 comprises a plurality of annular spring fingers 272 a, b, c within the cavity created by the locking mechanism. As illustrated in the first mounted position of Figure 5, these spring fingers 272 a, b, c engage a recess 239 located on the proximal end 226 of a main stem 237 of the connecting body main hub. The engagement of the spring fingers 272 a, b, c and the recess prevents the locking mechanism from moving in the distal direction prior to injection. This movable locking mechanism 268 is used to lock out the needle guard 290 after an injection has been made. That is, after the needle guard is first moved in a proximal direction and then returned in a distal direction under the force of the biasing member 270.

In this preferred arrangement, when the needle assembly 210 is initially mounted to the drug delivery device 212, the first piercing end 282 of the needle pierces the membrane 218 of the cartridge contained in the drug delivery device 212. The second piercing end 284 of the first needle 280 is illustrated as being substantially concealed from a user's view by way of the needle guard 290. Concealing the needle 280 helps to reduce needle anxiety that a patient may experience while also reducing a potential inadvertent needle stick.

Preferably, the needle guard 290 comprises a tubular shaped element and in a relaxed position, as illustrated in Figure 5, substantially conceals the needle 280. While substantially concealing this needle, the needle guard also helps to prevent inadvertent needle sticks. In Figure 5, this needle guard 290 is illustrated in an unlocked position. That is, during an injection step where a user initiates the injection, the needle guard 290 is free to be moved in a proximal direction or towards the drug delivery device. Preferably, the needle guard 290 comprises outwardly directed arms 296, 298 that are in sliding engagement with an inner surface 263 of the inner cavity 262 of the connecting body 224.

As illustrated in Figure 5, the module 210 is shown in a first mounted position on the drug delivery device 212. In this first position, the connecting body 224 is connected to a distal end of the drug delivery device 212. As illustrated, the drug delivery device comprises threads 213 for engagement with the connecting body 224. In one arrangement, the connecting body 224 may comprise a threaded connector to releasably engage these threads. However, in an alternative arrangement, the connecting body 224 may comprise a connector 230 comprising a form fit or snap fit arrangement or the like. In this manner, the module 210 may be connected to the drug delivery device 212 merely by sliding the module onto the distal end of the drug delivery device.

In this initial mounting position, the needle 280 is in fluid communication with the medicament contained in the cartridge. Where the drug delivery device 212 comprises a dose setter, a dose of the drug delivery device 212 may then be set using a dose setter 212 (see Figure 9) in the normal manner (e.g., by dialing out the appropriate number of units). Dispense of the medicament 216 may be achieved by subcutaneously injecting the medicaments via activation of a dose button on device 212. The dose button may be any triggering mechanism that causes the dose of the first medicament that was set by the dose setter to move distally towards the distal end of the device. In a preferred embodiment, the dose button is operably connected to a spindle that engages a piston in the primary reservoir of the first medicament.

During injection, the needle guard 290 is moved in a proximal direction 310 against a force created by the biasing member 270. As the needle guard moves proximally, its arms 296, 298 slide internally within the cavity 262 of the connecting body 224. Once the needle guard beveled edge 275 reaches the rib 274, the beveled edge slips around the rib so that the needle guard 290 picks up the movable locking feature 268. The medicament 216 may then be injected into an injection site by way of the needle 280.

After the injection, the drug delivery device and the module 210 are moved away from the injection site. Then, under the force of the biasing member 270, the needle guard 290 is forced in the distal direction 320. On being forced down or in the distal direction 320 by the force created by the biasing member 270, the needle guard 390 pulls the movable lockout member 268 distally.

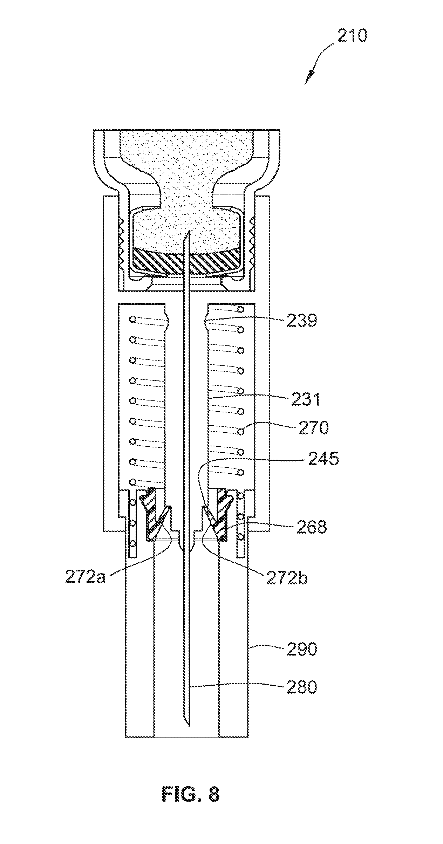

Figure 8 illustrates the module 210 with the needle guard 290 in a locked position. As illustrated, the annular ring fingers 272 a, b, c of the locking member 268 flex inwardly so to as to reside along a first recess 239 provided along the distal end of the main stem 237. As such, the annular ring fingers 272 prevent the needle guard 290 from moving in the proximal direction and therefore prevents a user from re-using the module.

Locking the needle guard 290 in the down position in this manner provides a number of beneficial features. First, it prevents a user from re-using a non-sterile medicated module. Second, the locked needle guard protects and substantially conceals the needle 280 and therefore reduces the risk of a potential inadvertent needle stick. In addition, by substantially concealing the needle 280, the locked needle guard 290 acts to reduce any potential need fear, needle phobia or needle anxiety that a patient may experience.

As discussed above with respect to Figure 1, the medicated module 10 is shown in a first mounted position on the drug delivery device 12. In this first position, the connecting body 24 is connected to a distal end of the drug delivery device 12. As illustrated in this arrangement, the drug delivery device comprises a conventional thread arrangement 13 for engagement with the connecting body 24. However, in an alternative arrangement, the connecting body 24 may comprise a connector 30 comprising a form fit or snap fit connector arrangement or the like. In this manner, the medicated module 10 may be connected to the drug delivery device 12 merely by sliding the medicated module onto the distal end of the drug delivery device.

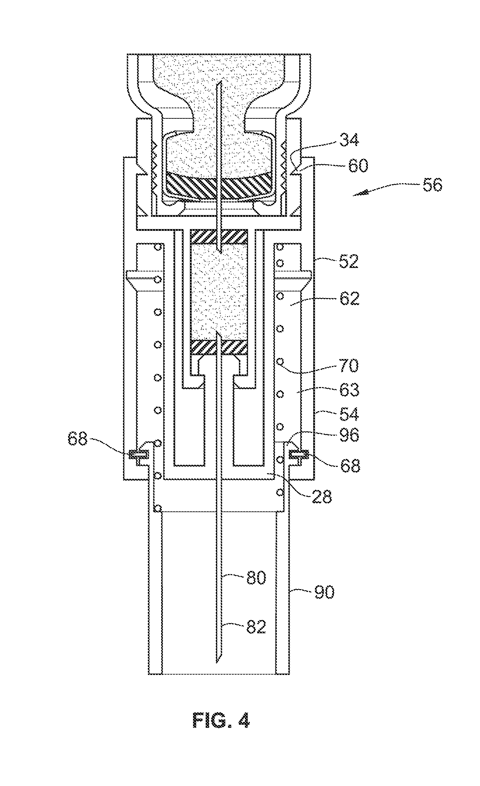

The connection or attachment between the medicated module (illustrated in Figures 1-4) as well as the non-medicated module (illustrated in Figures 5-8) may contain additional features, such as connectors, stops, splines, ribs, grooves, and the other similar mechanical design features, that ensure that only a specific medicated module or non-medicated module is only attachable to a complementary drug delivery device. That is, the specific needle assembly (i.e., a medicated module or a non-medicated module) could comprise certain mechanical features that are dedicated to work with only a specific/complementary dedicated drug delivery device. Such additional features would prevent the attachment of a non-appropriate medicated module to a non-matching injection device, such as a drug delivery device comprising a conventional coupling mechanism. In one preferred arrangement, the additional features, such as connectors, stops, splines, ribs, grooves, and the like mechanical design features may be integral to either the needle assembly or the drug delivery device or both. Alternatively, these additional features, such as connectors, stops, splines, ribs, grooves, and the like mechanical design features may be provided as an independent part or collection of parts that allow the needle assembly to properly interface with a convention drug delivery device, such as the device illustrated in Figure 9.

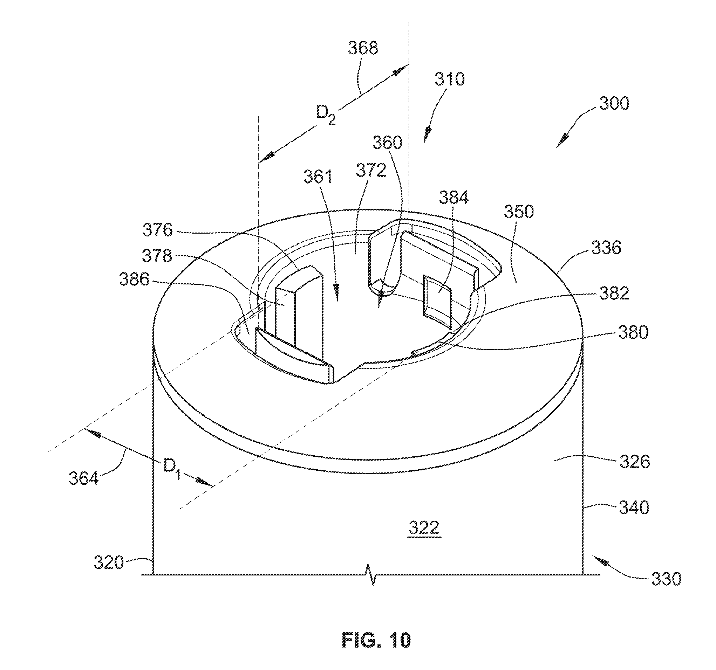

Figure 10 illustrates one example of such an additional feature in the form of a dedicated mechanical coupling 310 that can be used to dedicate a needle assembly 300, such as a medicated module, to a corresponding dedicated drug delivery device. As such, Figure 10 illustrates a perspective view of a first arrangement of a dedicated needle assembly 300 comprising a dedicated mechanical coupling 310.

The dedicated mechanical coupling 310 in this arrangement is integral with the dedicated needle assembly 300. For example, the needle assembly could take the form of the medicated module 10 illustrated in Figure 1 while replacing the threaded connector 30 with the dedicated mechanical coupling 310. However, in an alternative arrangement, the dedicated mechanical coupling 310 may comprise a separate component that may then be used to mate the medicated module illustrated in Figure 1 to the drug delivery device.

By dedicated needle assembly, it is meant that the needle assembly can only be properly mechanically coupled to a dedicated drug delivery device that is mechanically configured to administer a dose of medicament from a cartridge contained within the dedicated needle arrangement and cannot be used to properly administer a dose of a drug contained in an device that does not have the proper dedicated mechanical coupling.

In one preferred arrangement, this first needle assembly 300 may comprise a medicated module, such as the medicated module 10 illustrated in Figure 1. Alternatively, this first needle arrangement 300 may comprise a needle assembly that does not comprise a medicament, such as the non-medicated module 210 illustrated in Figure 5. In yet another alternative arrangement, the first needle assembly 300 may comprise a double ended needle assembly such as a conventional needle assembly 600 illustrated in Figure 20. In this alternative arrangement, an internal thread of this double ended needle assembly is replaced with the proposed dedicated mechanical coupling 310. However, as those of skill in the relevant art will recognize, the presently described, illustrated, and claimed dedicated needle assemblies may be used for other attachment arrangements, systems and arrangements comprising alternative dedicated mechanical couplings.

Returning to Figure 10, the dedicated needle assembly 300 comprises an outer connecting body 320 and this body may define a generally cylindrical shape 322. This outer connecting body 320 extends from a distal end 330 to a proximal end 336 of the needle assembly 300. An outer surface 340 of this connecting body 320 may comprise a generally smooth outer surface 326. An advantage of such a smooth surface is that it provides an area to provide a label or other similar source or contents identifier so that a user can identify the medicament contained within the needle assembly (if a medicament is provided).

The outer connecting body 320 further comprises a generally smooth proximal end surface 350 wherein this proximal end surface is mechanically configured to define an engaging cavity 360. One advantage of such a generally smooth proximal end surface 350 is that such a surface tends to reduce interference during the insertion of the drug delivery device and may also be used as an additional identifier of the contents/strength or concentration of the drug about to be attached to the primary delivery device. The engaging cavity 360 may have a generally circular shaped opening 361 and may be defined to essentially comprise two different engagement cavity diameters: a first engaging cavity diameter D1 364 and a second engaging cavity diameter D2 368. The second engaging cavity diameter D2 368 is larger or wider than first engaging cavity diameter D1 364. As will be explained in greater detail below, various mechanical elements that are provided along an inner wall 372 of this engaging cavity 360 are mechanically coded to receive a similarly dedicated distal end of a drug delivery device, similar to the drug delivery device illustrated in Figure 9.

However, the conventional threaded distal end of the drug delivery device illustrated in Figure 9 will need to modified to comprise a corresponding dedicated coupling mechanism that is coded to properly cooperate with the various mechanical elements of the engaging cavity 360. As just one example, the threaded distal end of this drug delivery device illustrated in Figure 9 can be altered by modifying the distal end of the cartridge holder so as to comprise a dedicated mechanical coupling.

Alternatively, the conventional threaded distal end of the drug delivery device can receive a separate component part that comprises the dedicated mechanical coupling. In such an arrangement, this separate component part is first coupled onto the distal end of the drug delivery device (e.g., rotated onto the conventional threaded distal end). Then, the distal end of the device having the separate component part is then inserted into the engaging cavity. One advantage of utilizing such a separate component part is that the drug delivery device used in such a dedicated needle assembly system may comprise a conventional threaded distal portion and will not need to be altered or modified. Such a dedicated needle assembly system can save production, manufacturing and storage costs that can necessary arise by having to manufacture and transport a large number of different drug delivery devices so as to provide the desired medicament and drug delivery device differentiation. This separate component part may be a releasably engaged or permanently attached at the point of manufacture. This would allow retrofitting of the dedicated features to an already established commercially available device. The latter action would help to ensure dedication by fixing the separate component part to the delivery device such that the user could not easily remove it, thus creating a dedicated delivery device without significant impact on an existing assembly line. The separate component part would simply be added as an additional component and could be added in a single extra assembly step.

Returning to Figure 10, the first engaging cavity diameter D1 364 has a diameter that is sized to be smaller than an outer diameter of a standard or a conventional Type A needle assembly. For example, comparing the needle assembly in Figure 10 with the conventional double ended needle assembly illustrated in Figure 20, the first engaging cavity diameter D1 364 comprises a diameter that is smaller than an outer diameter DType A 620 of the standard or conventional Type A threaded/cartridge holder interface illustrated in Figure 9. As such, a user is prevented from attaching the medicated module to a non-approved primary drug delivery device, such as the conventional device illustrated in Figure 9.

Returning to Figure 10, the engaging cavity 360 comprises an inner wall 372 located within the outer connecting body 320. A first and a second tongue or protrusion 376, 380 is provided along the inner wall 372. The first and second protrusions 376, 380 are offset 180 degrees from one another and situated across the first inner diameter D1 364. In addition, the engaging cavity 360 further comprises two recess features 384, 386 (only one of these two recess features 384 are illustrated in Figure 10) which are also provided along the inner wall 372. These recess features are also offset 180 degrees from one another and are situated across the second inner diameter D2 368.

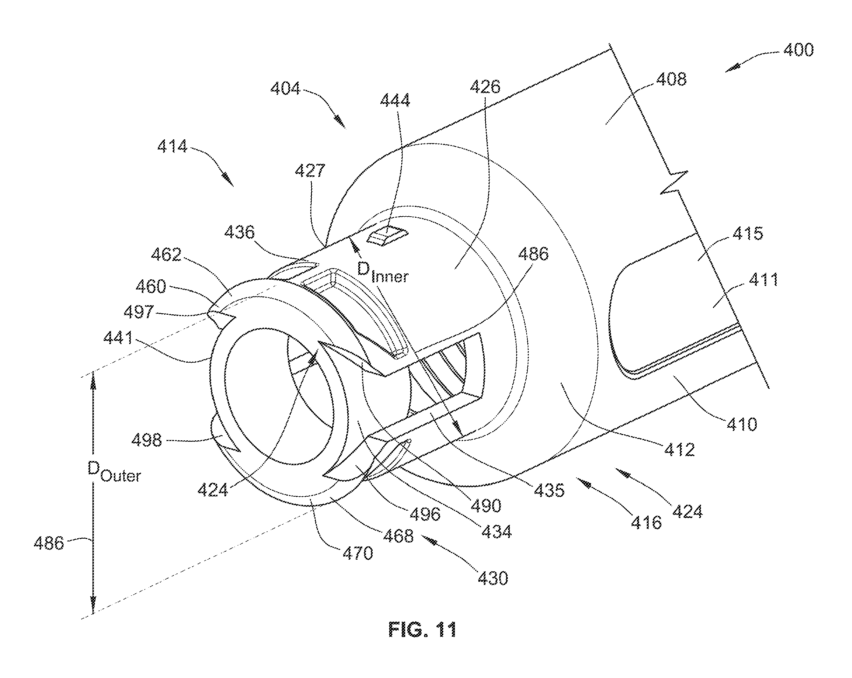

Figure 11 illustrates a distal end 404 of a dedicated drug delivery device 400 that is mechanically coded for use with a dedicated needle assembly, such as the dedicated needle assembly 300 illustrated in Figure 10. In one preferred arrangement, this dedicated drug delivery device 400 may be operated by a user to select a dose of a medicament and then administer this selected medicament dose in a similar fashion as the convention drug delivery illustrated in Figure 9. However, although the general mechanical operation between the two devices might be the same or similar, the distal end 404 of the dedicated drug delivery device 400 will comprise a modified distal end. With such a modified distal end 404, the dedicated drug delivery device 400 can only be mechanically and properly operatively connected to the dedicated needle assembly 300 illustrated in Figure 10.

Figure 11 illustrates only a portion of the dedicated drug delivery device 400 and illustrates a distal portion 408 of the dedicated cartridge holder 410 and a distal end 404 of the drug delivery device 400. The dedicated cartridge holder 410 houses or contains a cartridge or ampoule 411 and this cartridge 411 contains a medicament 415, such as long acting insulin. In one arrangement, the cartridge holder 410 may comprise a disposable cartridge holder. Alternatively, the cartridge holder 410 may comprise a reusable cartridge holder. By disposable cartridge holder, it is meant the cartridge holder may be obtained from the manufacturer preloaded with the cartridge containing the medicament 415 and the cartridge holder cannot be reloaded with new medicament (i.e., a new cartridge) after the initial medicament is exhausted. A reusable cartridge holder can allow the user to reload the holder with new medicament (i.e., a new cartridge).

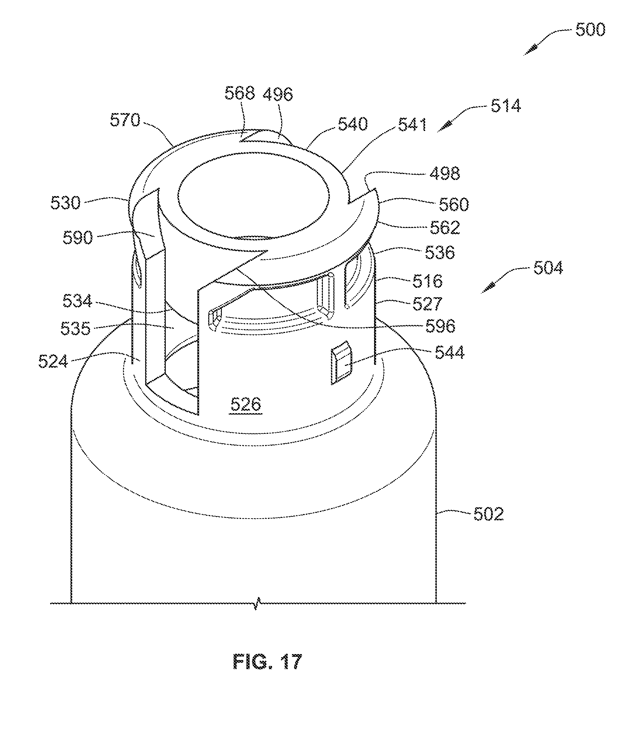

As can be seen in Figure 11, the distal end 404 of the dedicated drug delivery device 400 does not comprise a conventional threaded distal end (as the drug delivery device illustrated Figure 1). Rather, the distal end 404 comprises a dedicated mechanical coupling 414. This mechanical coupling 414 may be integral with the dedicated cartridge holder 410 (as illustrated in Figure 11). That is, the dedicated cartridge holder 410 along with the dedicated mechanical coupling 414 may be manufactured or molded as one integral component. Alternatively, the mechanical coupling 414 may comprise a separate component that can be attached to a threaded distal end of a conventional drug delivery device, such as the device illustrated in Figure 9. This alternative non-integral mechanical coupling arrangement is discussed in detail herein below with respect to Figures 17-19.

In a first arrangement, the illustrated dedicated mechanical coupling 414 comprises a generally cylindrical extension 416. This extension 416 extends from an extension proximal end 424 that is located near the cartridge holder 410 to an extension distal end 430. This generally cylindrical extension comprises a generally smooth first body portion 426 and a second body portion 436. The overall length of the first body portion 426 and the second body portion 436 can vary based on a depth of a complementary engaging cavity of a needle assembly, such as the engaging cavity 360 of needle assembly 300. However, in this illustrated arrangement, an overall length of the extension 416 can be selected so as to be properly inserted into the engaging cavity 360 of the needle assembly 300. The proximal end 424 may be located near a shoulder 412 of the cartridge holder 410 of the generally cylindrical extension 416.

As illustrated in Figure 11, the first body portion 426 of the generally cylindrical extension 416 extends from the proximal end towards the distal end of the generally cylindrical extension 416. The first body portion 426 comprises a generally smooth outer surface 427 and having an inner diameter Dinner 418.

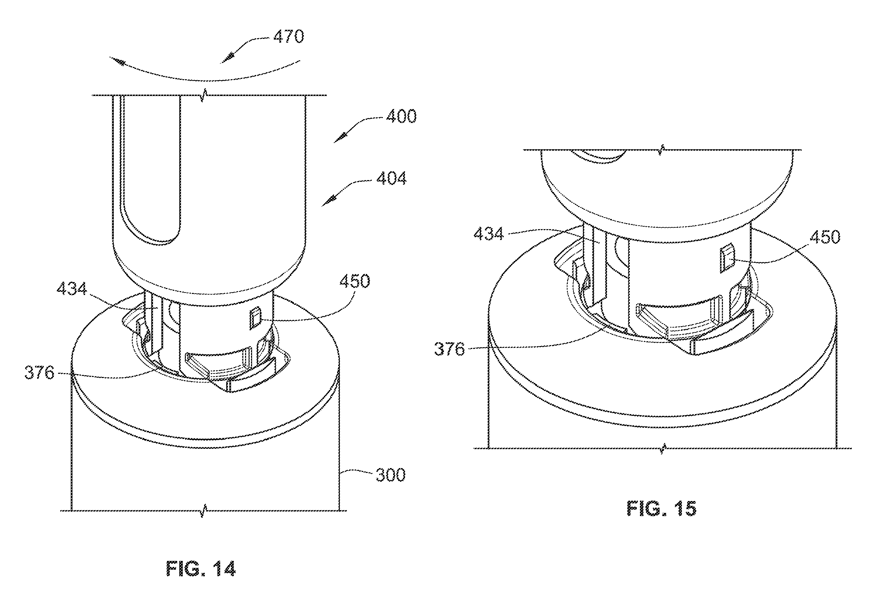

In one preferred arrangement, a plurality of bump features are provided along the outer surface 427. For example, in one illustrative arrangement, two bump features 444, 450 are positioned along the outer surface 427 and are preferably positioned 180 degrees apart from one another (second bump 450 illustrated in Figures 14-15). As illustrated, these bump features 444, 450 may be positioned along the cylindrical extension 416 so that when the dedicated mechanical coupling 414 is fully inserted into the engaging cavity 360 of the needle assembly 300 the first and second bump features 444, 450 interact or mate with the first 384 and second recess features, respectively. In one preferred arrangement, when a user properly inserts the distal end 404 of the drug delivery device 400 into the needle assembly 300 (Figure 10), the interaction between the first and second two bump features 444, 450 and the first and second recess features 384, 388 will provide a tactile and/or audible confirmation that the two components have been properly connected. One advantage of such a dedicated coupling mechanism is that it can provide both tactile and audio feedback to a user on full fitment provided by axially engaged bump features on cartridge holder clicking into recess features on medicated module. As those of skill in the art will recognize, alternative bump and ridge arrangements could also be utilized. As just one example, these features could be reversed where the bump features are provided by the needle assembly and the recess features are provided by the distal end of the drug delivery device.

The dedicated mechanical coupling 414 further comprises a first and a second upstand feature 460, 468. These upstand features 460, 468 may be located along a second body portion 436 of the cylindrical extension 416 and may extend from the proximal end 424 towards the distal end 430 of the cylindrical extension 416. At a most distal end, first upstand feature 460 comprises a first lip 462 wherein this lip 462 flares radially outward away from the smooth outer surface 427 of the first body portion 426. Second upstand feature 468 comprises a similar lip 470 that also flares radially outward away from the smooth outer surface 427 of the first body portion 426. As such, the outer most radially directed portions of lips 462, 470 define an outer diameter DOuter 486. In one arrangement, this outer diameter DOuter 486 is larger (or wider) than the inner diameter Dlnner 418 defined by the first body portion 426 of the cylindrical extension 426. In a preferred arrangement, this outer diameter DOuter 486 will be larger (or wider) than a diameter of a standard "Type A" screw thread needle assembly, such as the inner diameter DType A 620 of the conventional Type A double ended screw thread needle assembly illustrated in Figure 20. As such, a user will be prevented from attaching a conventional Type A double ended needle assembly 600 to the distal end 404 of the drug delivery device 400.

The dedicated mechanical coupling 414 further comprises a first and a second groove 434, 440. In one preferred groove arrangement, the first and second grooves 434, 440 are positioned 180 degrees apart from each other. Preferably, each groove 434, 440 defines a certain width. For example, first groove 434 may define a first width 435 and second groove 440 may define a second width 441. The first width 435 of the first groove 434 may or may not be equivalent to the second width 441 of the second groove 440. In one preferred arrangement, the first and second widths are generally equivalent. If the widths or rotational positioning of the ribs were to differ, this may be one such way the dedicated features could be coded within a family of different dedicated needle assemblies.

As illustrated, these groove widths 435, 441 may be generally equivalent to a width of the protrusions provided along the inner wall of the engaging cavity 360. For example, the first width 435 of the first groove 434 may be generally equivalent to a first width 378 of the first protrusion 376. Similarly, the width 441 of the second groove 440 may be generally equivalent to a width 382 of the second protrusion 380 of the dedicated needle assembly 300.

In addition, the dedicated mechanical coupling 414 may further comprise a first and a second chamfered edge 490, 496 provided near the distal opening of the first groove 434. The second groove 440 comprises a similar chamfer edge arrangement 497, 498. As discussed in greater detail below, one advantage of such a chamfered edge arrangement is that when inserting the distal end of cartridge holder into the needle assembly 300, these chamfer edges aid to guide the grooves into proper alignment with the receiving protrusions.

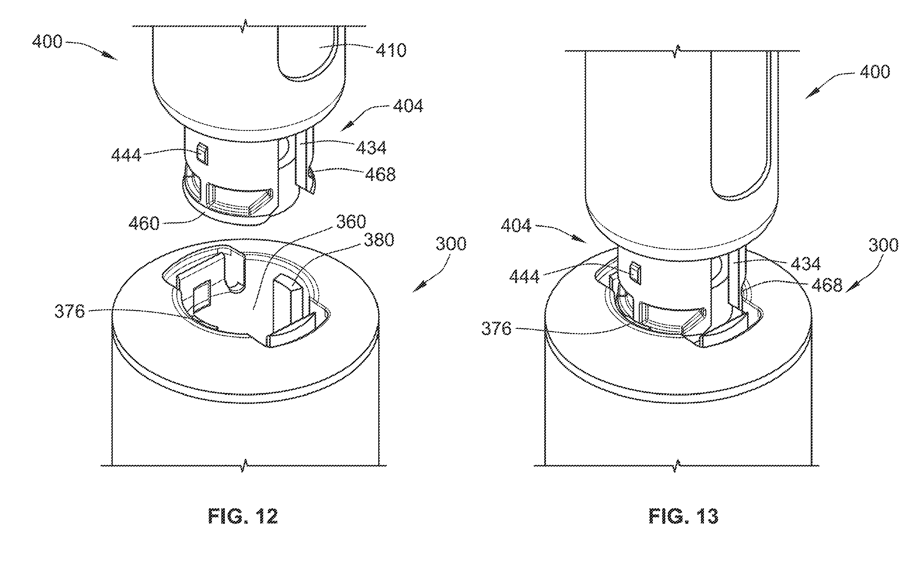

Figure 12 illustrates a perspective view of the dedicated drug delivery device 440 illustrated in Figure 11 just prior to being inserted into the engaging cavity 360 of the dedicated needle assembly 300 illustrated in Figure 10 where the distal end of the device is initially not aligned with the engaging cavity 360. Ordinarily, to insert the dedicated cartridge holder into this needle assembly, a user will typically hold the needle assembly 300 in one hand while holding the drug delivery device in the other hand while inserting the distal end 404 into the engaging cavity 360. During insertion, when the grooves 434, 440 on the cartridge holder 410 properly align with the protrusions of the needle assembly 300, the distal end of the device 404 can be properly inserted. Ordinarily, this connection action will comprise a purely axial motion on the part of the user.

However, during the process of inserting the drug delivery device into the needle assembly, there may be situations where the first and second grooves 434, 440 of the cartridge holder do not align with the first and second protrusions 376, 380 of the receiving cavity 360. As just one example, Figure 12 illustrates the situation where the protrusion and groove features are not properly aligned.

For example, Figure 12 illustrates the distal end 404 of the drug delivery device 400 in a first position. In this first position, the distal end 404 of the device attempts to enter the engaging cavity 360 in an axial direction. In this axial direction, initially, there is no rotation of the needle assembly 300 and no rotation of the drug delivery device 400. However, as illustrated in the close up view provided in Figure 13, the first groove 434 and the second groove 440 on the distal end 404 of the dedicated drug delivery device 400 are not properly aligned with the first protrusion 376 and second protrusion 380 of the engaging cavity 360, respectively.

Because the grooves 434, 440 of the cartridge holder are not aligned with the protrusions 376, 380, the outer diameter DOuter 486 defined by the first and second upstand features 460, 468 is blocked by the protrusions 376, 380 because the outer diameter DOuter 486 is wider than the first inner diameter D1 364 defined by the engaging cavity 360. As such, the distal end 404 of the drug delivery device 400 is prevented from axially entering the engaging cavity 360. Therefore, either the needle assembly 300 or the drug delivery device 400 must be rotated in order for the outer diameter DOuter 486 to be aligned with the larger second engaging cavity diameter D2 368 and so as to align the protrusions 376, 380 and the grooves 434, 440.

Figure 14 illustrates the situation where the protrusions and the grooves are now in alignment after the device 400 has been rotated in the direction of arrow 470 approximately 90 degrees. Figure 14 illustrates the protrusion 376 now in alignment with the groove 434. As illustrated, when inserted into the needle assembly 300, the drug delivery device 400 will rotate so as to align the protrusions with the grooves.

Once there is coarse alignment between the distal end 404 and the engaging cavity 360 is achieved, the chamfer edges of the up stand features 460, 468 will tend to assist the user in guiding these component parts together into an accurately controlled rotational alignment.

Figure 15 illustrates a close up view of the dedicated drug delivery device after the device has been rotated in the clockwise direction shown by arrow 470 (Figure 14) so as to align with the dedicated mechanical coupling of the dedicated needle assembly illustrated Figure 10. As can be seen from Figures 14 and 15, the first groove 434 is now in alignment with the first protrusion 376. Similarly, the wider diameter of DOuter 486 defined by the two up stand features 460, 468 is now also aligned with the larger diameter D2 368 of the engaging cavity 360. As the grooves and protrusions approach one another for a final alignment position, the chamfers on the first and second grooves will aid in mating the first groove with the first protrusion 376.

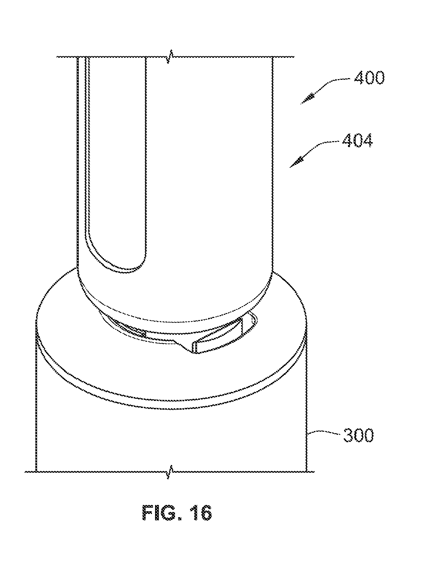

Figure 16 illustrates a perspective view of the distal end 404 of the dedicated drug delivery device 400 after it has been fully inserted into the dedicated needle assembly 300 illustrated in Figure 10. The needle assembly 300 and device 400 are now ready to administer a dose of the medicament 415 contained within the properly attached cartridge 411 contained within the drug delivery device 400. After a dose has been set and then administered, to remove the needle assembly 300 from the dedicated cartridge holder 410, a user merely needs to pull apart the two components. The retention feature between the dedicated needle assembly 300 and the dedicated drug delivery 400, in the illustrated arrangement, may be refined to optimise the forces required to attach and remove the needle assembly. As just one example, the flexible nature of the up stand features 460, 468 can be tuned to control this force to an acceptable level given various material selections. Similarly, the lead ins on each of the sides of the bump features 444, 450 can be tuned to result in different attachment and detachment forces should this be required.

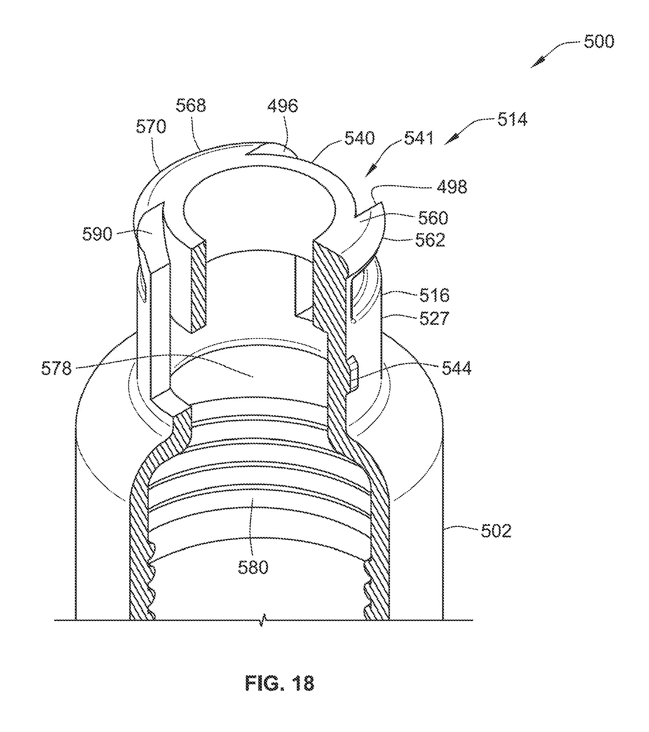

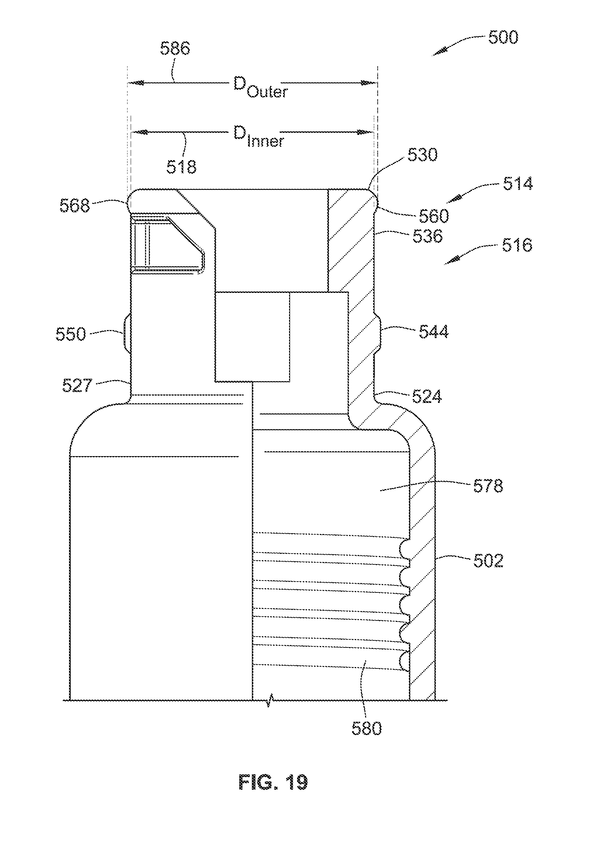

As discussed above, the dedicated mechanical coupling 310 may be integral with the dedicated needle assembly 300 or alternatively, the dedicated mechanical coupling 310 may comprise a separate component that is then used to interface between the medicated module, such as the medicated module illustrated in Figure 1, and the drug delivery device. For example, Figure 17 illustrates a perspective view of one arrangement of a dedicated cap 500 that may be used to dedicate a drug delivery device to the dedicated needle assembly illustrated Figure 10. Figure 18 illustrates a cut away view of the dedicated cap illustrated in Figure 17 and Figure 19 illustrates a partial sectional view of the dedicated cap illustrated in Figure 17.

Referring now to Figures 17-19, the dedicated cap 500 comprises a dedicated mechanical coupling 514 and this coupling comprises similar features as the dedicated mechanical coupling 414 of the drug delivery device 400 illustrated in Figure 11.

For example, Figure 17 illustrates a perspective view of a distal end 504 of a dedicated cap 500 that is mechanically coded for use with a dedicated needle assembly, such as the dedicated needle assembly 300 illustrated in Figure 10.

As can be seen from these Figures, the dedicated cap 500 comprises a dedicated mechanical coupling 514 comprising a main body 502 and a generally cylindrical extension 516 that extends from the main body 502. The cylindrical extension extends from an extension proximal end 524 to an extension distal end 530. This generally cylindrical extension comprises a generally smooth first body portion 526 and a second body portion 536. The overall length of the first body portion 526 and the second body portion 536 can vary based on a depth of a complementary engaging cavity of a needle assembly, such as the engaging cavity 360 of needle assembly 300. However, in this illustrated arrangement, an overall length of the extension 516 can be selected so as to be properly inserted into the engaging cavity 360 of the needle assembly 300.

As illustrated, the first body portion 526 of the generally cylindrical extension 516 extends from the proximal end towards the distal end of the generally cylindrical extension 516. The first body portion 526 comprises a first extension portion that comprises a generally smooth outer surface 527. This first extension portion 526 comprises an inner diameter Dlnner 518 (illustrated in Figure 19).

In one preferred arrangement, a plurality of bump features are provided along the outer surface 527. For example, in one illustrative arrangement, two bump features 544, 550 are positioned along the outer surface 527 and are preferably positioned 180 degrees apart from one another (both first and second bump features 544, 550 illustrated in Figure 19). As illustrated, these bump features 544, 550 may be positioned along the cylindrical extension 516 so that when the dedicated mechanical coupling 514 is fully inserted into the engaging cavity 360 of the needle assembly 300, the first and second bump features 544, 550 interact or mate with the first and second recess features 384, 388, respectively. In one preferred arrangement, when a user properly inserts the distal end 504 of the drug delivery device 400 into the needle assembly 300 (Figure 10), the interaction between the first and second bump features 544, 550 and the first and second recess features 384, 388 will be provide a tactile and/or audible confirmation that the two components have been properly connected.

The dedicated mechanical coupling 514 further comprises a first and a second upstand feature 560, 568 located along a second body portion 536 of the cylindrical extension 516 and may extend from the proximal end 524 towards the distal end 530 of the cylindrical extension 516. At a most distal end, first upstand feature 560 comprises a first lip 562 wherein this lip 562 flares radially outward away from the smooth outer surface 527 of the first body portion 526. Second upstand feature 568 comprises a similar lip arrangement 570. As such, the outer most radially directed portions of lips 562, 570 define an outer diameter DOuter 586 that is larger (or wider) than the inner diameter Dlnner 518 (see Figure 19). In a preferred arrangement, this outer diameter DOuter 586 will be larger (or wider) than an inner diameter of a standard "Type A" screw thread needle assembly, such as the outer diameter DType A 620 of the conventional Type A double ended screw thread needle assembly 600 illustrated in Figure 20. As such, a user will be prevented from attaching a conventional Type A double ended needle assembly onto the dedicated cap 500.

The dedicated mechanical coupling 514 further comprises a first and a second groove 534, 540 that are positioned 180 degrees apart from each other. Preferably, first groove 534 defines a first width 535 and the second groove 540 defines a second width 541. The first width 535 of the first groove 534 may or may not be equivalent to the second width 541 of the second groove 540. In one preferred arrangement, the first and second widths are generally equivalent.

These groove widths 535, 541 may be generally equivalent to a width of the protrusions provided along the inner wall of the engaging cavity 360. For example, the first width 535 of the first groove 534 may be generally equivalent to a first width 378 of the first protrusion 376. Similarly, the width 541 of the second groove 540 may be generally equivalent to a width 382 of the second protrusion 380 of the dedicated needle assembly 300.

In addition, the dedicated cap 514 may further comprise a first and a second chamfered edge 590, 596 provided near the distal opening of the first groove 534. The second groove 540 comprises a similar chamfer edge arrangement 496, 498. In addition, the dedicated cap comprises an inner surface 578 and on this surface, a connection mechanism 580 in the form of an internal screw thread is provided (illustrated in Figures 18 and 19). This screw thread allows the dedicated cap 500 to be threadedly coupled to the conventional threaded distal end of a drug delivery device, such as the device illustrated in Figure 9. Once connected to the drug delivery device, the dedicated cap 500 allows for the interconnection of the device to the needle assembly 300 in a similar fashion as described herein with respect to the dedicated mechanical coupling as described herein in detail. As those of skill in the art will recognize, alternative temporary and permanent connection mechanisms may also be used.

Figure 21 illustrates another example of a dedicated needle assembly. In particular, Figure 21 illustrates a dedicated mechanical coupling 652 that can be used to dedicate a needle assembly 650, such as a medicated module, to a corresponding dedicated drug delivery device. As such, Figure 21 illustrates a perspective view of a first arrangement of a dedicated needle assembly 650 comprising a dedicated mechanical coupling 652.

The dedicated mechanical coupling 652 in this arrangement is integral with the dedicated needle assembly 650. For example, the needle assembly could take the form of the medicated module 10 illustrated in Figure 1 while replacing the threaded connector 30 with the dedicated mechanical coupling 652. However, in an alternative arrangement, the dedicated mechanical coupling 652 may comprise a separate component that may then be used to mate the medicated module illustrated in Figure 1 to the drug delivery device. As mentioned above, by dedicated needle assembly, it is meant that the needle assembly can only be properly mechanically coupled to a dedicated drug delivery device that is mechanically configured to administer a dose of medicament from a cartridge contained within the dedicated needle arrangement and cannot be used to properly administer a dose of a drug contained in an device that does not have the proper dedicated mechanical coupling.

In the case where the dedicated mechanical coupling 652 comprises a separate component that may then be used to mate the medicated module illustrated in Figure 1 to the drug delivery device, the separate component may be a "step-down" type adaptor.

This would increase the operable length of the system in order to be able to fit the "step down" in diameter around a standard cartridge and would possibly mean that the proximal needle on the dedicated needle assembly may have to be longer in order to sufficiently penetrate the septum on the primary cartridge in the drug delivery device. This arrangement may be beneficial because it would mean that the coupling features on the dedicated needle assembly could be made much smaller than a standard type-A fitting (making it impossible to fit to a type-A threaded connection) while the secondary collar element (e.g., angled face) might be employed to block a standard type-A needle being fitted. The extra cannula length required as a result may also help prevent the proximal tip of the needle from penetrating the septum on the primary drug delivery device, thereby meaning no fluidic connection is made, and there is nothing to hold the type-A needle in place. Having a larger diameter (i.e. the additional component fits radially between the type-A thread and the exclusive connection features) is also feasible. While this may not result in an operational length increase, it may however enable the dedicated needle assembly to be in fluid communication to a standard type-A attachment (even if it is not mechanically coupled) as the coding features of the coupling would be larger than that of a type-A thread.

When such a separate component is used, the separate component may be substantially affixed to the cartridge holder at a stage before a user receives the device (e.g., during manufacture or final assembly). In a situation where the separate component is not substantially affixed to the cartridge holder, a user may elect to remove the separate component, and may then be able to affix a standard type-A needle to the drug delivery device. A user may elect to do this in an emergency situation. For instance, the user may need to affix a standard type-A needle to the drug delivery device when the user does not have a dedicated needle assembly available, but needs an emergency dose of the primary medicament.

In one preferred arrangement, this dedicated needle assembly 650 may comprise a medicated module, such as the medicated module 10 illustrated in Figure 1. Alternatively, this dedicated needle arrangement 300 may comprise a needle assembly that does not comprise a medicament, such as the non-medicated module 210 illustrated in Figure 5. In yet another alternative arrangement, the dedicated needle assembly 650 may comprise a double ended needle assembly such as a conventional needle assembly 600 illustrated in Figure 20. In this alternative arrangement, an internal thread of this double ended needle assembly is replaced with the proposed dedicated mechanical coupling 652. However, as those of skill in the relevant art will recognize, the presently described, illustrated, and claimed dedicated needle assemblies may be used for other attachment arrangements, systems and arrangements comprising alternative dedicated mechanical couplings.

Returning to Figure 21, the dedicated needle assembly 650 comprises an outer connecting body 654 and this body may define a generally cylindrical shape 656. This outer connecting body 654 extends from a distal end 658 to a proximal end 660 of the needle assembly 650. An outer surface 662 of this connecting body 654 may comprise a generally smooth outer surface 664. An advantage of such a smooth surface is that it provides an area to provide a label or other similar source or contents identifier so that a user can identify the medicament contained within the needle assembly (if a medicament is provided).

The dedicated mechanical coupling 652 is configured at the proximal end 660 of the connecting body. In this example, the dedicated mechanical coupling 652 includes a ring-shaped extension 668. As will be explained in greater detail below, the ring-shaped extension is mechanically coded to interact or mate with a similarly dedicated distal end of a drug delivery device, similar to the drug delivery device illustrated in Figure 9. However, the conventional threaded distal end of the drug delivery device illustrated in Figure 9 will need to be modified to comprise a corresponding dedicated coupling mechanism that is coded to properly cooperate with the various mechanical elements of the extension 668. As just one example, the threaded distal end of this drug delivery device illustrated in Figure 9 can be altered by modifying the distal end of the cartridge so as to comprise a dedicated mechanical coupling. Alternatively, the conventional threaded distal end of the drug delivery device can receive a separate component part that comprises the dedicated mechanical coupling, as discussed above with respect to Figure 10.

Returning to Figure 21, the extension 668 has an inner diameter D1 670 that is sized to be smaller than an outer diameter of a standard or a conventional Type A needle assembly. For example, comparing the needle assembly in Figure 21 with the conventional double ended needle assembly illustrated in Figure 20, the extension diameter D1 670 comprises a diameter that is smaller than an outer diameter DType A 620 of the standard or conventional Type A threaded/cartridge holder interface illustrated in Figure 9. As such, a user is prevented from attaching the medicated module to a non-approved primary drug delivery device, such as the conventional device illustrated in Figure 9.

Returning to Figure 21, the extension 668 may comprise at least one slit, such as slits 672, 674, and 676. ln this example, these slits are 120 degrees from one another. The arrangement and/or number of slits may provide for coding of a needle assembly, as will be discussed in more detail below. Further, the ring-shaped extension 668 is generally cylindrical. However, it should be understood that other shapes are possible as well. For instance, the ring-shaped extension 668 may be a polygonal-shaped extension, examples of which include but are not limited to a pentagon-shaped extension or an octagonal-shaped extension.

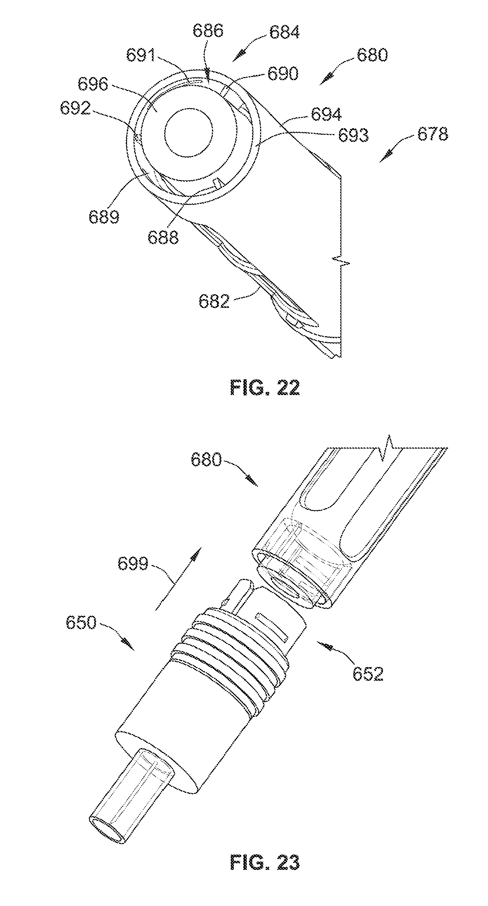

Figure 22 illustrates a distal end 678 of a dedicated drug delivery device 680 that is mechanically coded for use with a dedicated needle assembly, such as the dedicated needle assembly 650 illustrated in Figure 21. In one preferred arrangement, this dedicated drug delivery device 680 may be operated by a user to select a dose of a medicament and then administer this selected medicament dose in a similar fashion as the conventional drug delivery device illustrated in Figure 9. However, although the general mechanical operation between the two devices might be the same or similar, the distal end 678 of the dedicated drug delivery device 680 will comprise a modified distal end. With such a modified distal end 678, the dedicated drug delivery device 680 can only be mechanically and properly operatively connected to the dedicated needle assembly 650 illustrated in Figure 21.

Figure 22 illustrates only a portion of the dedicated drug delivery device 680 and specifically illustrates a distal portion of the dedicated cartridge holder 682. The dedicated cartridge holder 682 may house or contain a cartridge or ampoule and this cartridge may contain a medicament, such as long acting insulin. In one arrangement, the cartridge holder 682 may comprise a disposable cartridge holder. Alternatively, the cartridge holder 682 may comprise a reusable cartridge holder. By disposable cartridge holder, it is meant the cartridge holder may be obtained from the manufacturer preloaded with the cartridge containing the medicament and cartridge holder cannot be reloaded with new medicament (i.e., a new cartridge) after the initial medicament is exhausted. A reusable cartridge holder can allow the user to reload the holder with new medicament (i.e., a new cartridge).

As can be seen in Figure 22, the distal end 678 of the dedicated drug delivery device 680 does not comprise a conventional threaded distal end (as the drug delivery device illustrated Figure 1). Rather, the distal end 678 comprises a dedicated mechanical coupling 684. This mechanical coupling 684 may be integral with the dedicated cartridge holder 682 (as illustrated in Figure 22). That is, the dedicated cartridge holder 682 along with the dedicated mechanical coupling 684 may be manufactured or molded as one integral component. Alternatively, the mechanical coupling 684 may comprise a separate component that can be attached to a threaded distal end of a conventional drug delivery device, such as the device illustrated in Figure 9.

In the arrangement of Figure 22, the illustrated dedicated mechanical coupling 684 comprises an engaging ring-shaped cavity 686 that includes at least one rib feature, such as rib features 688, 690, and 692, disposed in the cavity 686. The end faces of the rib features may axially abut the end face of a standard "Type A" screw thread needle assembly, thereby preventing attachment of a standard needle assembly to the drug delivery device 680.

The engaging ring-shaped cavity 686 is a cavity that is formed between an outer edge 694 of the body of the cartridge holder and an inner tubular extension 696. The cavity 686 is sized and configured to correspond to the ring-shaped extension 668 of the needle assembly 650. The rib features 688, 690, and 692 have widths that are generally equivalent to the widths of the slits 672, 674, and 676. As shown in Figures 21, the slits 672, 674, and 676 are spaced evenly at 120 degree intervals and each generally have the same width. Similarly, the ribs 688, 690, and 692 are spaced evenly at 120 degree intervals and each generally have the same width. Thus, in this example, the needle assembly 650 could connect to the delivery device 680 in any of three orientations.

Additionally, coding of the needle assembly to the drug delivery device could be provided by the fit and location of these ribs and slots. If the widths or rotational positioning of the ribs were to differ, this may be one such way the dedicated features could be coded within a family of different dedicated needle assemblies. For instance, the location and/or widths may be varied so that only given needle assemblies may be connected to given drug delivery devices. For example, each slot (and corresponding rib) may have a separate width, such that the needle assembly will only fit in the drug delivery device in one orientation.

In one preferred arrangement, a plurality of bump features are provided along the outer surface 661 of the dedicated coupling 652. In the example depicted in Figure 21, the outer surface 661 has bump features 663, 665, and a third bump feature (not shown) on segment 667. The dedicated mechanical coupling 684 of the cartridge holder 682 includes corresponding recesses that are configured to receive the bump features. For example, the coupling 684 includes a first recess 689, a second recess 691, and a third recess (not shown) on segment 693. As illustrated, these bump features of the needle assembly may be positioned along the outer surface 661 so that when the dedicated mechanical coupling 652 is fully inserted into the engaging cavity 686 of the cartridge holder, the three bump features interact or mate with the three corresponding recess features, respectively. In one preferred arrangement, when a user properly inserts the proximal end of the needle assembly 650 into the distal end 678 of drug delivery device 680 (see Figure 23), the interaction between the bump features and the recess features will provide a tactile and/or audible confirmation that the two components have been properly connected. One advantage of such a dedicated coupling mechanism is that it can provide both tactile and audio feedback to a user on full fitment provided by axially engaged bump features on cartridge holder clicking into recess features on medicated module. As those of skill in the art will recognize, alternative bump and recess arrangements could also be utilized. As just one example, these features could be reversed where the bump features are provided by the distal end of the drug delivery device and the recess features are provided by the needle assembly.

In addition, the dedicated mechanical coupling 668 may further comprise chamfered edges provided near the distal opening of the slits 672, 674, and 676. One advantage of such a chamfered edge arrangement is that when inserting the distal end 678 of cartridge holder 682 into the needle assembly 650, these chamfered edges aid to guide the ribs into proper alignment with the receiving slits.

Figure 23 illustrates a perspective view of the dedicated drug delivery device 680 illustrated in Figure 22 just prior to being connected to the needle assembly 650 illustrated in Figure 21. Ordinarily, to insert the dedicated cartridge holder into this needle assembly, a user will typically hold the needle assembly 650 in one hand while holding the drug delivery device 680 in the other hand while inserting the proximal end of the needle assembly into the engaging cavity 686. During insertion, when the slots 672, 674, and 676 and ribs 688, 690, and 692 properly align, the needle assembly and drug delivery device can be connected together. Ordinarily, this connection action will comprise a purely axial motion on the part of the user. For example, as shown in Figure 23, the needle assembly may be pushed onto the delivery device in axial direction 699. In order to detach the needle assembly 650 from the drug delivery device 680, a user may pull the needle assembly off the drug delivery device in an axial direction opposite axial direction 699.

During the process of inserting the needle assembly 650 into the drug delivery device 680, there may be situations where the ribs and slits are not properly aligned. In this case, features (e.g., grip details, surface treatments, indicia or like features) may be present on the respective components to provide visual assistance / guidance such that the user can align the slots and ribs. As mentioned above, the chamfered edges may assist the user to properly align the ribs and slits.

As discussed above, the dedicated mechanical coupling 652 may be integral with the dedicated needle assembly 650 or alternatively, the dedicated mechanical coupling 652 may comprise a separate component that is then used to interface between the medicated module, such as the medicated module illustrated in Figure 1, and the drug delivery device.

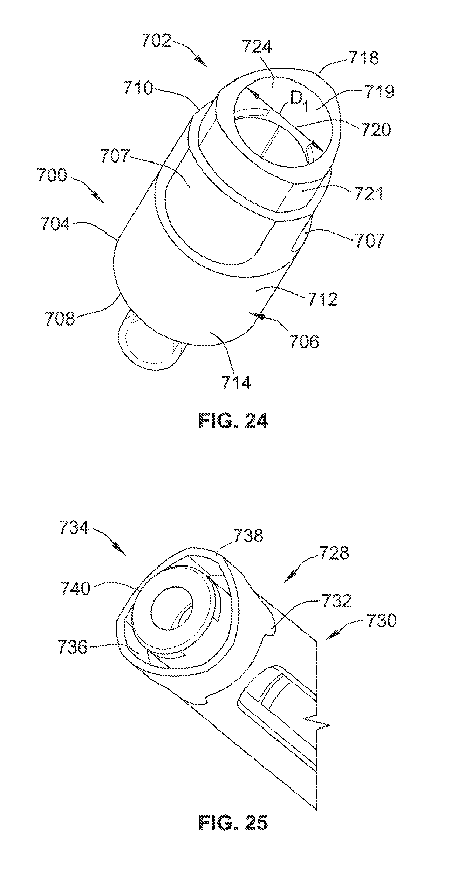

Figure 24 illustrates yet another example of a dedicated needle assembly. In particular, Figure 24 illustrates a dedicated mechanical coupling 702 that can be used to dedicate a needle assembly 700, such as a medicated module, to a corresponding dedicated drug delivery device. The dedicated mechanical coupling 702 in this arrangement is integral with the dedicated needle assembly 700. For example, the needle assembly could take the form of the medicated module 10 illustrated in Figure 1 while replacing the threaded connector 30 with the dedicated mechanical coupling 702. However, similar to the needle assemblies discussed above, in an alternative arrangement, the dedicated mechanical coupling 702 may comprise a separate component that may then be used to mate the medicated module illustrated in Figure 1 to the drug delivery device.

In one preferred arrangement, this dedicated needle assembly 700 may comprise a medicated module, such as the medicated module 10 illustrated in Figure 1. Alternatively, this dedicated needle arrangement 700 may comprise a needle assembly that does not comprise a medicament, such as the non-medicated module 210 illustrated in Figure 5. In yet another alternative arrangement, the dedicated needle assembly 700 may comprise a double ended needle assembly such as a conventional needle assembly 600 illustrated in Figure 20. In this alternative arrangement, an internal thread of this double ended needle assembly is replaced with the proposed dedicated mechanical coupling 652. However, as those of skill in the relevant art will recognize, the presently described, illustrated, and claimed dedicated needle assemblies may be used for other attachment arrangements, systems and arrangements comprising alternative dedicated mechanical couplings.

Returning to Figure 24, the dedicated needle assembly 700 comprises an outer connecting body 704 and this body may define a generally cylindrical shape 706 having ergonomic impressions 707. This outer connecting body 704 extends from a distal end 708 to a proximal end 710 of the needle assembly 700. Similar to the needle assembly 650, an outer surface 712 of this connecting body 704 may comprise a generally smooth outer surface 714. An advantage of such a smooth surface is that it provides an area to provide a label or other similar source or contents identifier so that a user can identify the medicament contained within the needle assembly (if a medicament is provided).

The dedicated mechanical coupling 702 is configured at the proximal end 710 of the connecting body 704. In this example, the dedicated mechanical couple 702 includes a ring-shaped extension 718. This ring-shaped extension comprises an inner edge 719 and an outer edge 721. This outer edge 721 is a non-circular outer edge. It should be understood that numerous non-circular shapes are possible. For example, numerous polygonal shapes are possible.

Similar to the example of Figure 21, the ring-shaped extension 718 is mechanically coded to interact or mate with a similarly dedicated distal end of a drug delivery device, similar to the drug delivery device illustrated in Figure 9. However, the conventional threaded distal end of the drug delivery device illustrated in Figure 9 will need to be modified to comprise a corresponding dedicated coupling mechanism that is coded to properly cooperate with the various mechanical elements of the extension 718. As just one example, the threaded distal end of this drug delivery device illustrated in Figure 9 can be altered by modifying the distal end of the cartridge holder so as to comprise a dedicated mechanical coupling. Alternatively, the conventional threaded distal end of the drug delivery device can receive a separate component part that comprises the dedicated mechanical coupling, as discussed about with respect to Figure 10. Returning to Figure 24, the extension 718 has an inner diameter D1 720 that is sized to be smaller than an outer diameter of a standard or a conventional Type A needle assembly. For example, comparing the needle assembly in Figure 24 with the conventional double ended needle assembly illustrated in Figure 20, the extension diameter D1 720 comprises a diameter that is smaller than an outer diameter DType A 620 of the standard or conventional Type A threaded/cartridge holder interface illustrated in Figure 9. As such, a user is prevented from attaching the medicated module 700 to a non-approved primary drug delivery device, such as the conventional device illustrated in Figure 9.

Returning to Figure 24, the extension 718 is configured to engage with a dedicated mechanical coupling of a dedicated drug delivery device. Figure 25 illustrates a distal end 728 of a dedicated drug delivery device 730 that is mechanically coded for use with a dedicated needle assembly, such as the dedicated needle assembly 700 illustrated in Figure 24. In one preferred arrangement, this dedicated drug delivery device 730 may be operated by a user to select a dose of a medicament and then administer this selected medicament dose in a similar fashion as the conventional drug delivery device illustrated in Figure 9. However, although the general mechanical operation between the two devices might be the same or similar, the distal end 728 of the dedicated drug delivery device 730 will comprise a modified distal end. With such a modified distal end 728, the dedicated drug delivery device 730 can only be mechanically and properly operatively connected to the dedicated needle assembly 700 illustrated in Figure 24.

Figure 25 illustrates only a portion of the dedicated drug delivery device 730 and specifically illustrates a distal portion of the dedicated cartridge holder 732. The dedicated cartridge holder 732 may house or contain a cartridge or ampoule and this cartridge may contain a medicament, such as long acting insulin. In one arrangement, the cartridge holder 732 may comprise a disposable cartridge holder. Alternatively, the cartridge holder 732 may comprise a reusable cartridge holder.

As can be seen in Figure 25, the distal end 728 of the dedicated drug delivery device 730 does not comprise a conventional threaded distal end (as the drug delivery device illustrated Figure 1). Rather, the distal end 728 comprises a dedicated mechanical coupling 734. This mechanical coupling 734 may be integral with the dedicated cartridge holder 732 (as illustrated in Figure 25). That is, the dedicated cartridge holder 732 along with the dedicated mechanical coupling 734 may be manufactured or molded as one integral component. Alternatively, the mechanical coupling 734 may comprise a separate component that can be attached to a threaded distal end of a conventional drug delivery device, such as the device illustrated in Figure 9.

In the arrangement of Figure 25, the illustrated dedicated mechanical coupling 734 comprises an engaging ring-shaped cavity 736. The ring-shaped cavity 736 has a shape corresponding to the ring-shaped extension 718. The cavity 736 is a cavity that is formed between an outer edge 738 and an inner tubular extension 740. In an example, the outer wall or edge 738 abuts the end face of a standard "Type A" screw thread needle assembly, thereby preventing attachment of a standard needle assembly to the drug delivery device 730.

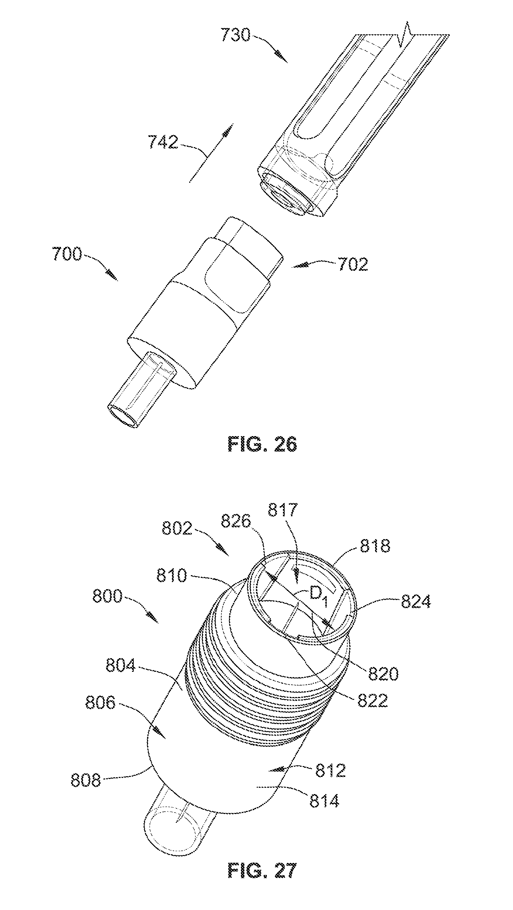

Further, similar to the example of Figure 21, in one preferred arrangement, a plurality of bump features are provided on the dedicated mechanical coupling 702. In this example, the plurality of bump features are disposed on an inner surface 724 of the coupling 702. The dedicated mechanical coupling 734 of the cartridge holder 732 includes corresponding recesses that are configured to receive the bump features. As illustrated, these bump features of the needle assembly may be positioned along the inner surface 724 so that when the dedicated mechanical coupling 702 is fully inserted into the engaging cavity 736 of the cartridge holder, the plurality of bump features interact or mate with the plurality of corresponding recess features, respectively. Once again, In one preferred arrangement, when a user properly inserts the proximal end of the needle assembly 700 into the distal end of drug delivery device 730 (see Figure 26), the interaction between the bump features and the recess features will provide a tactile and/or audible confirmation that the two components have been properly connected. As those of skill in the art will recognize, alternative bump and ridge arrangements could also be utilized. As just one example, these features could be reversed where the bump features are provided by the distal end of the drug delivery device and the recess features are provided by the needle assembly.

Figure 26 illustrates a perspective view of the dedicated drug delivery device 730 illustrated in Figure 25 just prior to being connected to the needle assembly 700 illustrated in Figure 24. In order to insert, the non-circular extension needs to be aligned with the corresponding non-circular cavity. Ordinarily, after alignment, this connection action will comprise a purely axial motion on the part of the user. For example, as shown in Figure 23, the needle assembly may be pushed onto the delivery device in axial direction 742. In order to detach the needle assembly 700 from the drug delivery device 730, a user may pull the needle assembly off the drug delivery device in an axial direction opposite axial direction 742.

Beneficially, if the non-circular shape of various needle assemblies and dedicated delivery devices were to differ, this may be one such way the dedicated features could be coded within a family of different dedicated needle assemblies. Further, the non-circular form of the extension 718 and cavity 734 beneficially serves to prevent rotation of the needle assembly 700 when the needle assembly 700 is connected to the drug delivery device 730.

As discussed above, the dedicated mechanical coupling 702 may be integral with the dedicated needle assembly 700 or alternatively, the dedicated mechanical coupling 702 may comprise a separate component that is then used to interface between the medicated module, such as the medicated module illustrated in Figure 1, and the drug delivery device.

Figure 27 illustrates another example of a dedicated needle assembly. In particular, Figure 27 illustrates a dedicated mechanical coupling 802 that can be used to dedicate a needle assembly 800, such as a medicated module, to a corresponding dedicated drug delivery device. As such, Figure 27 illustrates a perspective view of a first arrangement of a dedicated needle assembly 800 comprising a dedicated mechanical coupling 802.

The dedicated mechanical coupling 802 in this arrangement is integral with the dedicated needle assembly 800. For example, the needle assembly could take the form of the medicated module 10 illustrated in Figure 1 while replacing the threaded connector 30 with the dedicated mechanical coupling 802. However, in an alternative arrangement, the dedicated mechanical coupling 802 may comprise a separate component that may then be used to mate the medicated module illustrated in Figure 1 to the drug delivery device.

In one preferred arrangement, this dedicated needle assembly 800 may comprise a medicated module, such as the medicated module 10 illustrated in Figure 1. Alternatively, this dedicated needle arrangement 800 may comprise a needle assembly that does not comprise a medicament, such as the non-medicated module 210 illustrated in Figure 5. In yet another alternative arrangement, the dedicated needle assembly 800 may comprise a double ended needle assembly such as a conventional needle assembly 600 illustrated in Figure 20. In this alternative arrangement, an internal thread of this double ended needle assembly is replaced with the proposed dedicated mechanical coupling 802. However, as those of skill in the relevant art will recognize, the presently described, illustrated, and claimed dedicated needle assemblies may be used for other attachment arrangements, systems and arrangements comprising alternative dedicated mechanical couplings.

Returning to Figure 27, the dedicated needle assembly 800 comprises an outer connecting body 804 and this body may define a generally cylindrical shape 806. This outer connecting body 804 extends from a distal end 808 to a proximal end 810 of the needle assembly 800. An outer surface 812 of this connecting body 804 may comprise a generally smooth outer surface 814. An advantage of such a smooth surface is that it provides an area to provide a label or other similar source or contents identifier so that a user can identify the medicament contained within the needle assembly (if a medicament is provided).

The dedicated mechanical coupling 802 is configured at the proximal end 810 of the connecting body. In this example, the dedicated mechanical coupling 802 includes a ring-shaped extension 818. Similar to the examples discussed above, the ring-shaped extension 818 is mechanically coded to interact or mate with a similarly dedicated distal end of a drug delivery device.

The extension 818 has an inner diameter D1 820 that is sized to be smaller than an outer diameter of a standard or a conventional Type A needle assembly. For example, comparing the needle assembly in Figure 27 with the conventional double ended needle assembly illustrated in Figure 20, the extension diameter D1 820 comprises a diameter that is smaller than an outer diameter DType A 620 of the standard or conventional Type A threaded/cartridge holder interface illustrated in Figure 9. As such, a user is prevented from attaching the medicated module to a non-approved primary drug delivery device, such as the conventional device illustrated in Figure 9.

The extension 818 may comprise at least one indentation or slot that runs axially down an inner surface 817 of the extension 818. For example, the example depicted in Figure 27 includes three indentations 822, 824, and 826. In this example, these indentations are approximately 120 degrees from one another. The arrangement and/or number of indentations may provide for coding of a needle assembly, as will be discussed in more detail below. Further, the ring-shaped extension 818 is generally cylindrical. However, it should be understood that other shapes are possible as well. For instance, the extension may be a polygonal-shaped extension, examples of which include but are not limited to a pentagon-shaped extension or an octagonal-shaped extension.

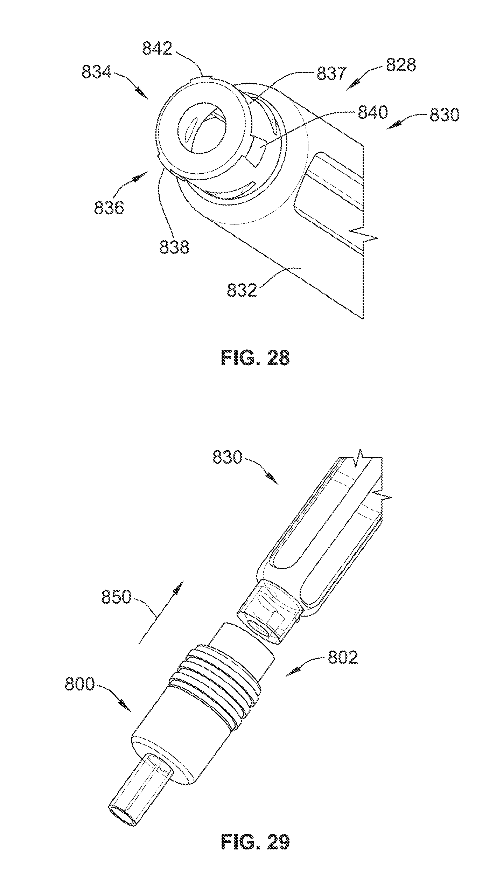

Figure 28 illustrates a distal end 828 of a dedicated drug delivery device 830 that is mechanically coded for use with a dedicated needle assembly, such as the dedicated needle assembly 800 illustrated in Figure 27. In one preferred arrangement, this dedicated drug delivery device 830 may be operated by a user to select a dose of a medicament and then administer this selected medicament dose in a similar fashion as the convention drug delivery illustrated in Figure 9. However, although the general mechanical operation between the two devices might be the same or similar, the distal end 828 of the dedicated drug delivery device 830 will comprise a modified distal end. With such a modified distal end 828, the dedicated drug delivery device 830 can only be mechanically and properly operatively connected to the dedicated needle assembly 800 illustrated in Figure 21.

Figure 28 illustrates only a portion of the dedicated drug delivery device 830 and specifically illustrates a distal portion of the dedicated cartridge holder 832. The dedicated cartridge holder 832 may house or contain a cartridge or ampoule and this cartridge may contain a medicament, such as long acting insulin. In one arrangement, the cartridge holder 832 may comprise a disposable cartridge holder. Alternatively, the cartridge holder 832 may comprise a reusable cartridge holder.

As can be seen in Figure 28, the distal end 828 of the dedicated drug delivery device 830 does not comprise a conventional threaded distal end (as the drug delivery device illustrated Figure 1). Rather, the distal end 828 comprises a dedicated mechanical coupling 834. This mechanical coupling 834 may be integral with the dedicated cartridge holder 832 (as illustrated in Figure 28). That is, the dedicated cartridge holder 832 along with the dedicated mechanical coupling 834 may be manufactured or molded as one integral component. Alternatively, the mechanical coupling 834 may comprise a separate component that can be attached to a threaded distal end of a conventional drug delivery device, such as the device illustrated in Figure 9.

In the arrangement of Figure 28, the illustrated dedicated mechanical coupling 834 comprises an engaging ring-shaped extension 836. This ring-shaped extension 836 includes at least one protrusion or rib feature that correspond to the indentations or slots of an intended needle assembly. These protrusions are disposed on an outer surface 837 of the extension 836. In this example, the extension 836 includes three protrusions 838, 840, and 842 that are approximately 120 degrees apart. The outer diameter of the protrusions is larger than an inner diameter of a standard "Type A" screw thread needle assembly, thereby preventing attachment of a standard needle assembly to the drug delivery device 830.

The engaging ring-shaped extension 836 is sized and configured to correspond to the ring-shaped extension 818. The protrusions 838, 840, and 842 have widths that are generally equivalent to the widths of the indentations 822, 824, and 826. As shown in Figures 27, the indentations 822, 824, and 826 are spaced evenly at 120 degree intervals and each generally have the same width. Similarly, the protrusions 838, 840, and 842 are spaced evenly at 120 degree intervals and each generally have the same width. Thus, in this example, the needle assembly could connect to the delivery device in any of three orientations.

Additionally, coding of the needle assembly to the drug delivery device could be provided by the fit and location of the protrusions and indentations. If the widths or rotational positioning of the ribs were to differ, this may be one such way the dedicated features could be coded within a family of different dedicated needle assemblies. For instance, the location and/or widths may be varied so that only given needle assemblies may be connected to given drug delivery devices. For example, each indentation (and corresponding protrusion) may have a separate width, such that the needle assembly will only fit in the drug delivery device in one orientation. It should be understood that in some examples the needle assembly may comprise the protrusions, while the cartridge holder comprises the indentations. In such an example, the cartridge holder may still be configured to not attach to a standard Type A needle.

Similar to the other arrangements discussed above, the needle assembly 800 and drug delivery device 830 may include a system of bump features and corresponding recess features. In one preferred arrangement, when a user properly inserts the proximal end of the needle assembly 800 into the distal end of drug delivery device 830 (see Figure 29), the interaction between the bump features and the recess features will provide a tactile and/or audible confirmation that the two components have been properly connected.

Figure 29 illustrates a perspective view of the dedicated drug delivery device 830 illustrated in Figure 28 just prior to being connected to the needle assembly 800 illustrated in Figure 27. Ordinarily, to insert the dedicated cartridge holder into this needle assembly, a user will typically hold the needle assembly 800 in one hand while holding the drug delivery device in the other hand while forcing the engaging ring 818 over the engaging extension 834. In an example, the needle assembly 800 may still be held in a sterile secondary packaging at this point. In such a case, features may be present on the secondary packaging to assist a user with correctly aligning the needle assembly to the cartridge holder prior to being fully inserted (i.e., such that the protrusions and channels are aligned). During insertion, when the indentations 822, 824, and 826 and corresponding protrusions 838, 840, and 842 properly align, the needle assembly and drug delivery device can be connected. Ordinarily, this connection action will comprise a purely axial motion on the part of the user. For example, as shown in Figure 23, the needle assembly may be pushed onto the delivery device in axial direction 850. However, during the process of inserting the drug delivery device into the needle assembly, there may be situations where the protrusions and indentations are not properly aligned. In this case, the user can align the protrusions and indentations. In order to detach the needle assembly 800 from the drug delivery device 830, a user may pull the needle assembly off the drug delivery device in an axial direction opposite axial direction 850.

As discussed above, the dedicated mechanical coupling 802 may be integral with the dedicated needle assembly 800 or alternatively, the dedicated mechanical coupling 802 may comprise a separate component that is then used to interface between the medicated module, such as the medicated module illustrated in Figure 1, and the drug delivery device. Further, in another example, the ring-shaped extension of the needle assembly may include a dedicated mechanical coupling that comprises a ring-shaped extension configured to form a releasable connection to an engaging ring-shaped cavity of a dedicated drug delivery device (rather than a ring-shaped extension). In such a case, an inner edge of the ring-shaped extension may include at least one recess (or, alternatively, at least one protrusion), and an inner edge of the engaging ring-shaped cavity may include at least one corresponding protrusion (or, alternatively, at least one corresponding indentation). Still further, in other examples, the dedicated coupling may comprise a mix between protrusions and indentations, and the drug delivery device may include a mix of corresponding protrusions and indentations.

As mentioned above, the dedicated mechanical coupling may comprise a separate component that may then be used to mate the medicated module to the primary drug delivery device. There are many possible benefits associated with using a dedicated connector rather than modifying the cartridge holder so that the cartridge holder itself includes the dedicated mechanical coupling. For instance, a dedicated connector may beneficially offer manufacturing flexibility. In an example, the primary drug delivery device may be supplied with (i) a standard Type-A interface (e.g. for the delivery of a single drug formulation) as well as (ii) in a format requiring an exclusive interface (e.g. for combination drug delivery in conjunction with a medicated module). The use of an interface connector component may enable the manufacturing company to rapidly adjust the relative number of units produced of each type, e.g. in order to respond to market demands, by simply altering the number of units to which the interface connector is added. Further, the use of an interface connector component may mean that the main filling and assembly lines for the primary drug delivery device may not need to be adapted to accommodate multiple cartridge holder variants as the connector fitment (e.g., if undertaken at the manufacturing facility rather than by the user) can be implemented as an additional 'cell' downstream from the main assembly process.

As another benefit, dedicated connectors may be potentially retro-fittable on existing drug delivery devices. The interface connectors could be provided to existing users of re-usable devices in order to enable the existing users to use such devices with an approved secondary device (e.g., a dedicated medicated module).

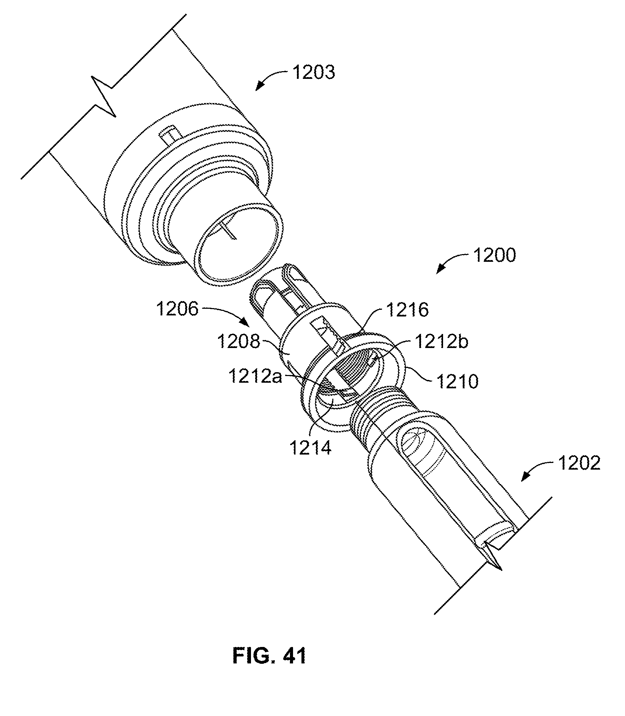

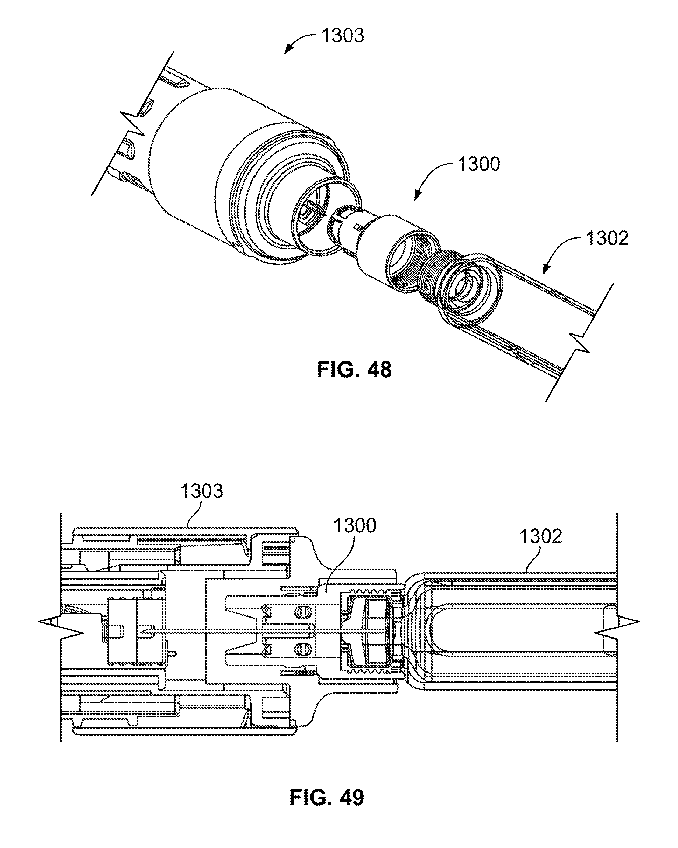

As yet another example, dedicated connectors may offer flexibility advantages for a user. For example, if the connector is configured to be releasably attached to a drug delivery device (e.g. screwed on and off), then a patient or other user is able to choose whether or not to utilize the primary drug delivery device on its own (e.g., with a conventional standard Type-A needle assembly) or in combination with a dedicated secondary device (e.g., a medicated module). Examples where removal and attachment action is repeatable (such as the example discussed below with respect to Figure 49) may be useful for scenarios such as split-dosing and 'top-up' dosing of the primary medicament while still preserving some of the safety advantages of having the dedicated connector interface. Alternatively, as in the example discussed below with respect to Figure 41, the connector may be configured such that it is removable by a patient, but removable in a manner that requires a deliberate and conscious secondary action, and removable in a manner that prevents or discourages the connector or another connector from being re-attached. The intention of such embodiments would be to provide a patient or other user with an 'emergency option' for use in instances where, for example a medicated module (which may only be available via prescription) is not available (e.g. the user may have run out, or there is a problem with the one they are trying to use) but they have access to a standard Type-A needle assembly (widely available, non-prescription). In the specific example of the delivery of the combination dose of insulin (via the primary drug delivery device) plus GLP-1 (via the medicated module), this would at least mean that a patient is able to receive the insulin resulting in at least a partially effective dose of medication, rather than no dose at all.

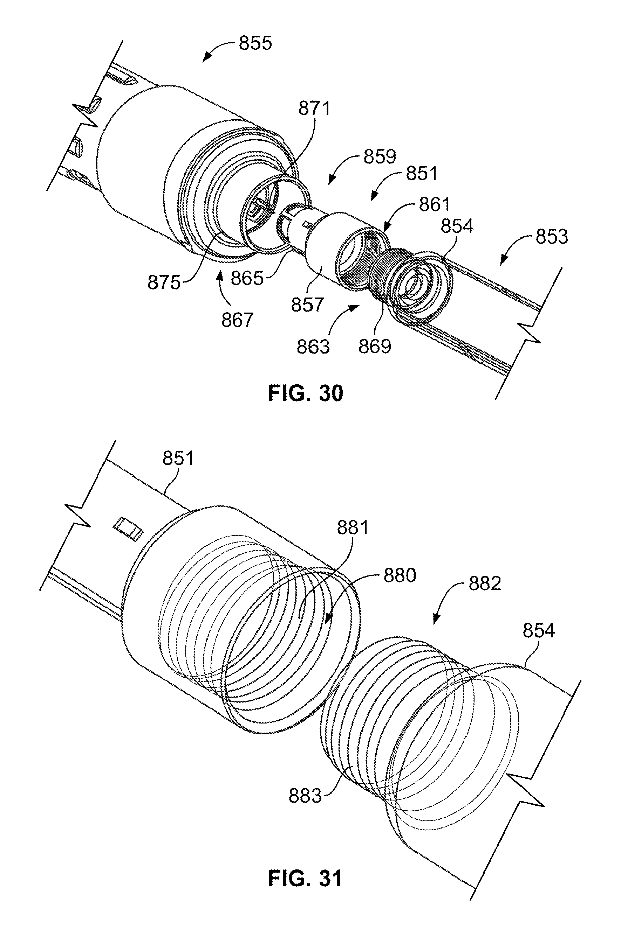

Figures 30-51 generally depict various examples of connectors in accordance with Applicants' proposed concept. Figures 30-51 also generally depict the distal end of drug delivery devices (and in particular the distal ends of the cartridge holders of the drug delivery devices) and dedicated needle assemblies (e.g., medicated modules) configured to be used with the various example dedicated connectors. Generally, a dedicated connector in accordance with Applicants' proposed concept includes a main body extending from a distal end to a proximal end. The proximal end of the main body is configured for attaching to a distal end of a drug delivery device, such as a distal end of a cartridge holder of the drug delivery device. The connector also includes a dedicated mechanical coupling configured at the distal end of the main body, wherein the dedicated mechanical coupling is configured to form a connection to a proximal end of a dedicated needle assembly, such as a dedicated medicated module. Such a connector may be configured to connect to a conventional mechanical coupling of a drug delivery device. However, in other examples, the connector may be configured to connect to a modified (i.e., non-conventional) mechanical coupling of a drug delivery device. Various example connectors are discussed in greater detail below.

In an example of Applicants' proposed concept, the dedicated connector may be designed so that the connector is permanently fixed to the drug delivery device, wherein the drug delivery device has a conventional mechanical coupling. For example, the drug delivery device may include a cartridge holder that includes a conventional Type-A screw thread. Beneficially, a dedicated connector configured to attach to a conventional mechanical coupling does not require manufacturing of a non-standard cartridge holder for a drug delivery device.

Figure 30 depicts such an example dedicated connector. In particular, Figure 30 depicts a dedicated connector 851 for connecting a drug delivery device 853 having cartridge holder 854 to a dedicated needle assembly 855. Similar to the examples above, the needle assembly may be a medicated module. The dedicated connector 851 includes a main body 857 extending from a distal end 859 to a proximal end 861. The proximal end 861 of the main body 857 is configured for attaching to a distal end 863 of drug delivery device 853. The dedicated connector 851 also includes a dedicated mechanical coupling 865 configured at the distal end 859 of the main body 857. The dedicated mechanical coupling 865 is configured to form a connection to a proximal end 867 of dedicated needle assembly 855. In an example, the connection may be a non-releasable (i.e., permanent) connection; however, in another example, the connection may be a releasable (i.e., non-permanent) connection.

In the example of Figure 30, the dedicated connector 851 may be permanently mounted on the conventional (i.e., standard Type-A) cartridge holder mount 869. For instance, the connector 851 may be screwed onto the conventional screw thread mount 869 and bonded into place. This connection and bonding may occur, for example, during an assembly process for the drug delivery device 853.

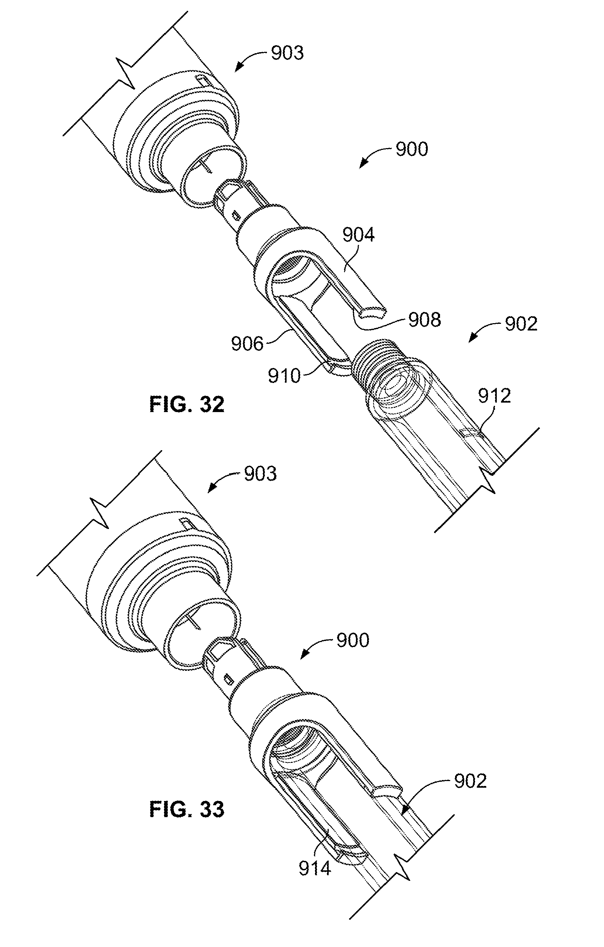

In the example of Figure 30, the connector 851 acts as a "step-down" connector. This connector 851 increases the operable length of the system in order to be able to fit the "step down" in diameter around a standard cartridge-holder mechanical coupling. In this example, this means that the proximal needle on the dedicated needle assembly 855 may have to be longer than that of a needle in a needle assembly (e.g., medicated module) not configured to be used with a connector. The needle may need to be longer in order to sufficiently penetrate the septum on the primary cartridge in the drug delivery device 853. Thus, the proximal needle 871 of the dedicated needle assembly 855 may be extended (e.g., compared to the length of a needle of a conventional needle assembly) to allow penetration of the septum through the connector 851 and the cartridge holder 853. Alternatively, the proximal needle 871 may be proved on the connector 851, for example on a connector hub. Note that although the example of Figure 30 (and the other examples shown in Figures 32-50) depicts a step-down connector, in other examples, the dedicated connector could be a step-up connector. That is, the diameter of the dedicated mechanical coupling of the dedicated connector may be greater than the diameter of the mechanical coupling of the drug delivery device.

The dedicated needle assembly 855 may include a needle shroud, such as needle shroud 875. The needle shroud 875 may protect users from needle-stick-related injuries that may be caused by the needle 871 of the needle assembly. This shroud 875 is particularly beneficial due to the longer-than-conventional needle 871 of the dedicated medicated module 855. The longer-than-conventional needle 871 without the needle shroud 875 could potentially increase the risk of needle-stick-related injuries.

Beneficially, when the connector 851 is disposed on drug delivery device 853, a needle assembly with a conventional mechanical coupling (e.g., a standard type-A needle assembly) cannot engage with the connector 851 and drug delivery device 853. Only a dedicated needle assembly intended to be connected to the dedicated mechanical coupling of connector 851—such as dedicated needle assembly 855—can be operably engaged with the connector 851. Similarly, the dedicated needle assembly 855 cannot operably engage a conventional mechanical standard type-A cartridge holder.

As mentioned above, the dedicated connector 851 may be permanently bonded to the drug delivery device 853. Figure 31 illustrates potential bonding surfaces 880, 882 on the dedicated connector 851 and the cartridge holder 854. In this example, the bonding surface 880 is located on the threads 881 of the proximal end of the dedicated connector 851, and the bonding surface 882 is located on the threads 883 of the cartridge holder 854.

Adhesive, mechanical, and/or thermal bonding between threads or planar surfaces may be applied during assembly to ensure that connector 851 can be (i) fitted to the standard type-A cartridge holder 854 during assembly but not (ii) removed from that cartridge holder 854 by the user. In an example, specialist polyolefin primers with ethyl cyanoacrylate or pre-treatment may be used for bonding to PolyPropyle (PP) cartridge holders. Conventional cyanoacrylates or methylene chloride may be suitable for bonding to PolyCarbonate (PC) cartridge holders. In another example, alternative treatments, such as those with lower toxicity, may also be used. Still further, friction, ultrasonic or laser welding may be used to bond the dedicated connector 851 to the cartridge holder 854; however, such methods may likely require a sub-assembly to avoid possible heat contamination of drug cartridge 854.

The dedicated mechanical coupling 865 of dedicated connector 851 may be configured in various ways. For example, the dedicated mechanical coupling 865 may be configured such that the dedicated needle assembly 855 is pushed on the dedicated connector when the dedicated needle assembly is attached to the drug delivery device. The example dedicated connectors shown in Figures 30-51 are configured in this way. In another example, however, the dedicated mechanical coupling 865 may be configured such that the dedicated needle assembly is twisted onto the dedicated connector when the dedicated needle assembly is attached to the drug delivery device. In yet another example, the dedicated mechanical coupling 865 may be configured such that the dedicated needle assembly is latched onto the dedicated connector when the dedicated needle assembly is attached to the drug delivery device. Other example configurations and attachment techniques are possible as well.

Similarly, the dedicated mechanical coupling 865 may be configured such that the dedicated needle assembly 855 is pulled off the dedicated connector when the dedicated needle assembly is detached from the dedicated drug delivery device. In another example, the dedicated mechanical coupling 865 may be configured such that the dedicated needle assembly is twisted off the dedicated connector when the dedicated needle assembly is detached from the dedicated drug delivery device. In yet another example, the dedicated mechanical coupling 865 may be configured such that the dedicated needle assembly is unlatched from the dedicated connector when the dedicated needle assembly is detached from the dedicated drug delivery device. Other example configurations and detachment techniques are possible as well.

Additional dedicated connectors are described with reference to Figures 32-51. These dedicated connectors discussed below are similar in many respects to the example of Figure 30, and thus these dedicated connectors are not described in as great of detail. It should be understood, however, that many of the possibilities and permutations discussed above with respect to dedicated connector 851 are possible as well.

Figure 32 depicts another example dedicated connector that can be permanently mounted to a drug delivery device, where the drug delivery device has a conventional mechanical coupling. Similar to the example of Figure 30, the drug delivery device may include a cartridge holder that includes a conventional Type-A screw thread.

This example utilizes additional bonding surfaces and an additional mechanical interface. In particular, in this example, the dedicated connector 900 may be screwed and snapped onto the cartridge holder 902. This connection may occur during the manufacturing and assembly process. A dedicated needle assembly, such as dedicated medicated module 903, may then be connected to the connector 900 and cartridge holder 902.

As mentioned above, this dedicated connector 900 is similar in many respects to dedicated connector 851; however, the dedicated connector 900 also includes arm features 904, 906 protruding from the proximal end of the main body of the dedicated connector 900. The arm features may include an engagement feature for engaging with a corresponding engagement feature of the cartridge holder. For example, arm feature 904 may include a clip feature 908, and arm feature 906 may include a clip feature 910. The clip features may be configured to interact with corresponding clip features, such as corresponding clip features 912 (see Figure 32) and 914 (see Figure 33). Figure 33 illustrates the dedicated connector 900 fully twisted and snapped onto cartridge holder 902.

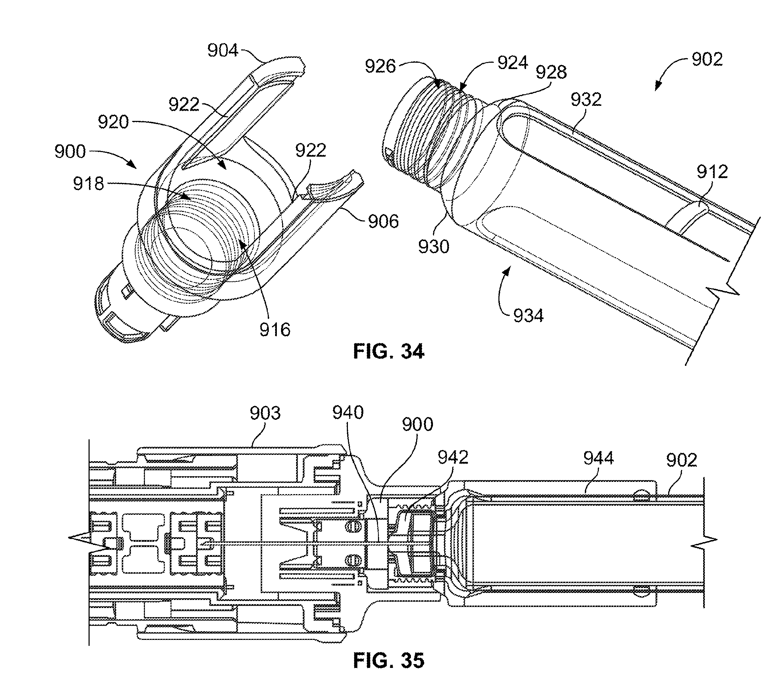

The dedicated connector 900 beneficially has increased bonding surface area— compared to the bonding surface area of dedicated connector 851—for bonding the dedicated connector 900 to the cartridge holder 902. The increased bonding surface area may beneficially make the connector 900 even more difficult to remove from the cartridge holder 902. Figure 34 depicts example bonding surfaces. Similar to the example dedicated connector 851 of Figure 30, dedicated connector 900 may have bonding surface 918 located on the threads 916 of the proximal end of the main body of the dedicated connector 900. In addition, dedicated connector 900 may have bonding surface 920 located on the inner surface of the proximal end, as well as bonding surface 922 located on the inner surface of the arms 904, 906. The cartridge holder 902 has corresponding bonding surfaces. In particular, the cartridge holder has bonding surface 924 located on the threads 926 of the cartridge holder 902. The cartridge holder 900 also has bonding surface 928 located on the neck 930 of the cartridge holder 902, as well as bonding surface 932 located on the outer surface of the holder and the clip feature 912 of the holder. Although not shown, similar (opposing) features on the other side 934 of the cartridge holder 902 may also be used for a bonding surface.

Figure 35 depicts a detailed cross-sectional view of the medicated module 903 attached to the dedicated connector 900 and cartridge holder 902. As shown, the needle 940 from the medicated module 903 is an extended needle that is long enough to penetrate the septum 942 of the cartridge 944 disposed in cartridge holder 902. Again, as previously described, the needle 940 may be configured within an inner hub of the connector 900. As mentioned above with reference to the dedicated needle assembly of Figure 30, the extended needle 940 may be longer than a needle of a conventional needle assembly

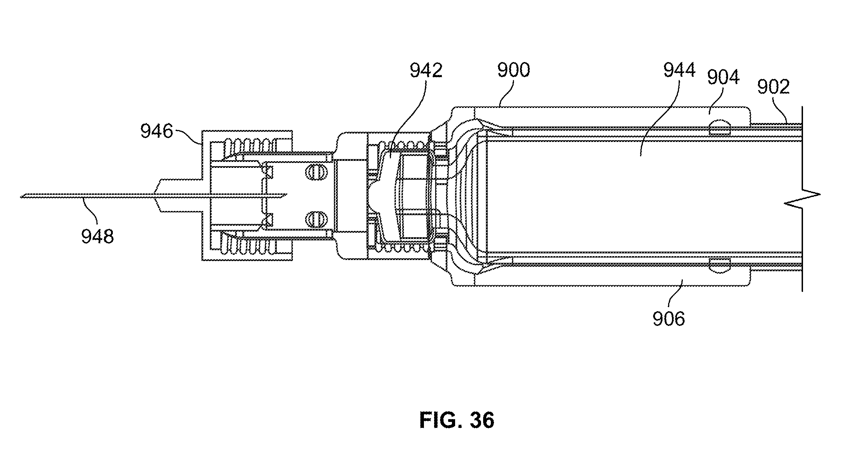

Figure 36 depicts a cross-sectional view of a conventional needle assembly 946 (i.e., a Type-A needle assembly) that a user may attempt to connect to the dedicated connector 900 and cartridge holder 902. As evident from the illustration, the conventional needle assembly 946 will not properly connect to the dedicated connector 900 and cartridge holder 902. First, the proximal portion of the conventional needle 948 is not long enough to pierce the septum 942 of cartridge 944, and thus fluid engagement between the needle 948 and the cartridge 944 is not possible. Second, the conventional mechanical coupling does not interface properly with the dedicated mechanical coupling 900, and thus a user would be unable to operably connect the conventional needle assembly 946 onto the dedicated connector 900.

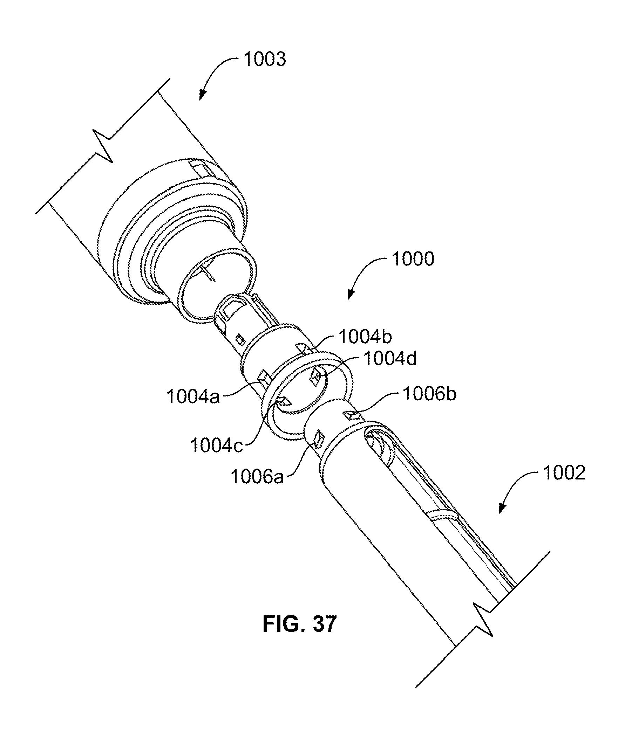

Another example dedicated connector is depicted in Figure 37. In particular, Figure 37 depicts a dedicated connector 1000 that can be permanently mounted onto a modified (i.e., non-conventional) cartridge holder 1002. A dedicated needle assembly, such as dedicated medicated module 1003, may then be connected to the connector 1000 and cartridge holder 1002. This dedicated connector 1000 is similar in many respects to dedicated connectors 851 and 900; however, the dedicated connector 1000 is slightly modified, as it is intended to connect to a modified cartridge holder 1002. In addition, the cartridge holder 1002 is similar to a conventional cartridge holder; however, rather than having a conventional screw thread at the distal end, the cartridge holder 1002 addionally comprises engagement features at the distal end.

The dedicated connector 1000 includes at least one engagement feature, where each of the at least one engagement feature is configured for interacting with a respective corresponding engagement feature disposed on the cartridge holder 1002. In particular, the dedicated connector 1000 in the example of Figure 37 includes four engagement features 1004a-d, each of which can engage with a corresponding engagement feature on the cartridge holder 1002. Figure 37 shows two of the four corresponding engagement features 1006a-b. In this example, the engagement features are snap engagement features. That is, the engagement features 1004a-d are configured to snap over and engage (e.g., permanently) with the corresponding engagement features.

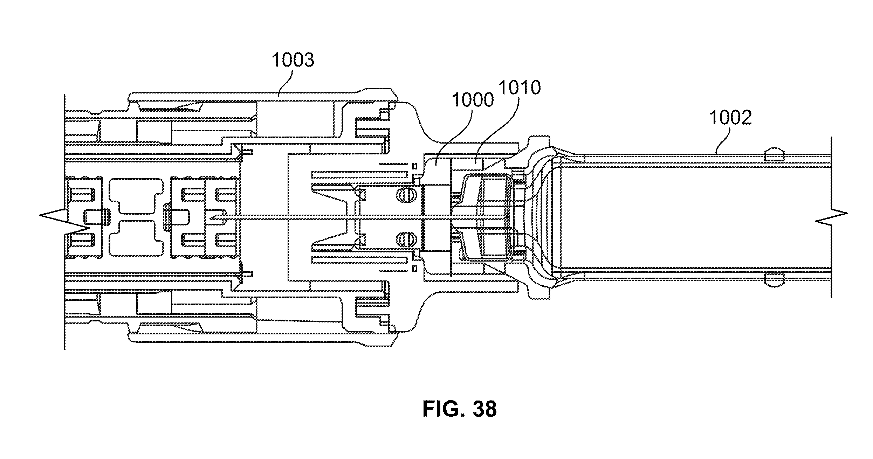

In one example, the engagement features 1004a-d may be snap apertures or snap recesses, and the corresponding engagement features 1006a-b may be corresponding bumps that are sized to fit in and engage the apertures or recesses. The engagement features 1004a-d may be disposed on an inner edge of a flexible wall 1010 (see Figure 38) of the dedicated connector 1000. Further, the flexible wall 1010 may be configured to deflect outward during attachment so that the each of the at least one snap engagement feature can deflect over and engage with the corresponding engagement feature when the dedicated connector 1000 is connected to the cartridge holder 1002. As such, the snap apertures could flex over the engagement bumps when the dedicated connector is attached to the cartridge holder, and the snap apertures would then snap over the engagement bumps. The apertures and bumps may preferably also be configured such that it would not be possible to remove the dedicated connector from the holder after the snap apertures snapped over the bumps. In another example, the snap apertures or a snap recess could be disposed on the cartridge holder 1002, and the engagement bumps could be disposed on the dedicated connector 1000. Note that although the dedicated connector engagement features are primarily described as axial snap engagement features, other types of engagement features are possible as well, such as radial snap features, i.e., features that prevent rotation in the unscrewing direction. Further, although in this example four engagement features are shown, more or fewer engagement features are also possible. Further, the system may be configured such that the number of snap apertures provided is greater than the number of engagement features.

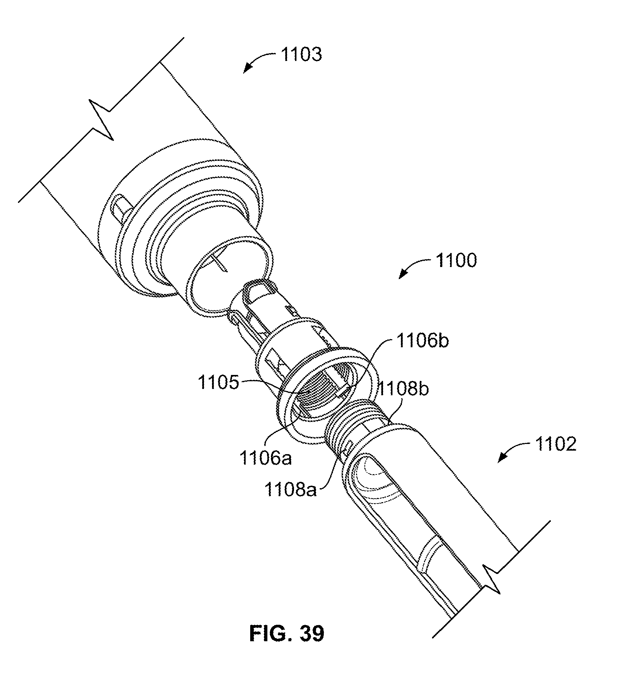

In another dedicated-connector example, a dedicated connector such as dedicated connector 1000 may be modified to also include an internal thread. In particular, Figure 39 depicts a dedicated connector 1100 having an internal thread, where the dedicated connector 1100 can be permanently mounted onto a modified (i.e., non-conventional) cartridge holder 1102. A dedicated needle assembly, such as dedicated medicated module 1103, may then be connected to the connector 1100 and cartridge holder 1102.

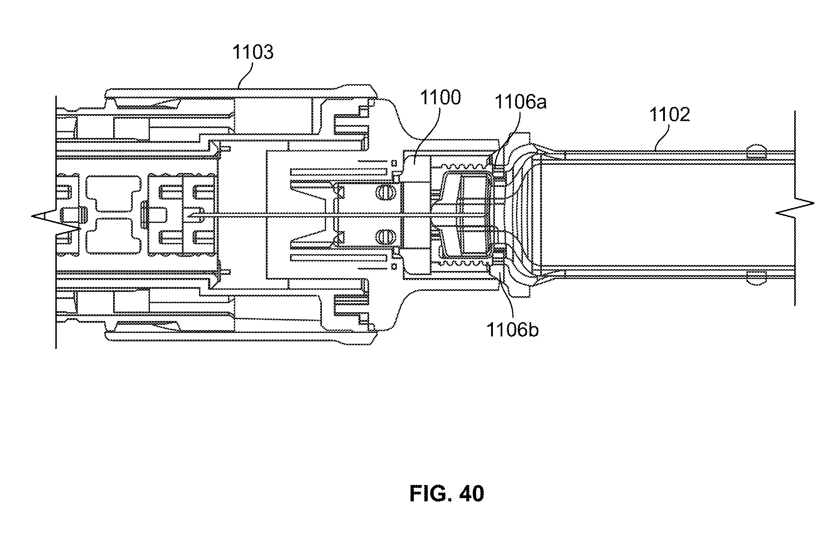

This dedicated connector 1100 may include an internal thread 1105 and at least one engagement feature, such as engagement features 1106a-b. In this example, the engagement features 1106a-b are flexible snap arms that are configured to engage with snap apertures 1108a-b. In this example, the dedicated connector 1100 may be screwed onto the cartridge holder at any time (e.g., during assembly or by a user); however, once the flexible snap arms 1106a-b have engaged with the snap apertures 1108a-b, the connector 1100 cannot be removed from the cartridge holder 1102 without the application of excessive / abusive torque. Figure 40 depicts a cross-sectional view of the connector 1100 attached to the cartridge holder 1102 and the dedicated needle assembly 1103. As can be seen in Figure 40, the snap arms 1106a-b are engaged, and thus the connector 1100 cannot be removed from the cartridge holder 1102 without the application of excessive / abusive torque. As with the prior embodiments, the proximal needle may be provided on a hub of the connector 1100.

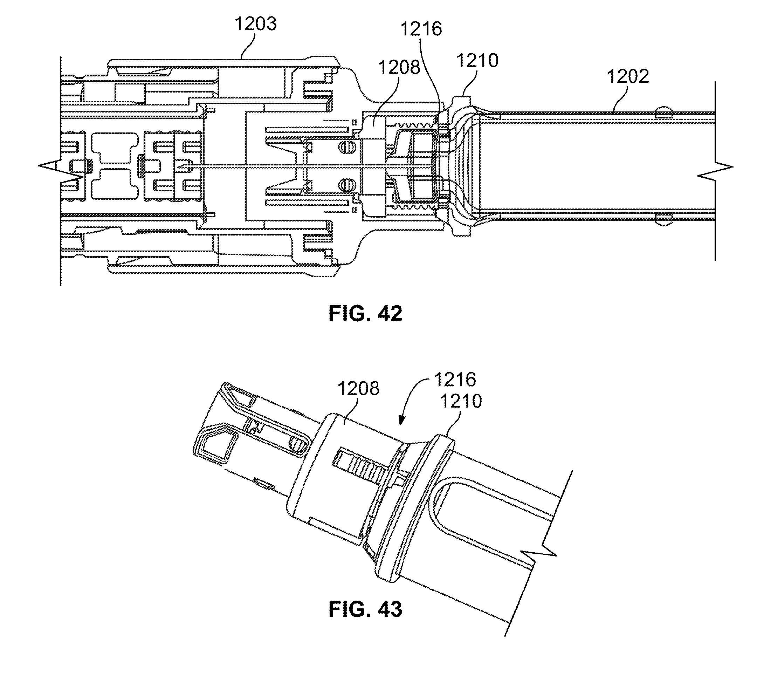

In another dedicated-connector example, the dedicated connector may be a connector that is configured to be a semi-permanent connector. Figure 41 depicts an example of such a dedicated connector and Figure 42 depicts a cross-sectional view of the connector. This connector is similar in many respects to dedicated connector 1100. However, the dedicated connector 1200 comprises frangible elements configured to allow the connector to be semi-permanently attached.

In particular, Figure 41 depicts a dedicated connector 1200 that can be semi-permanently mounted onto a modified (i.e., non-conventional) cartridge holder 1202. A dedicated needle assembly, such as dedicated medicated module 1203, may then be connected to the combined connector 1200 and cartridge holder 1202.

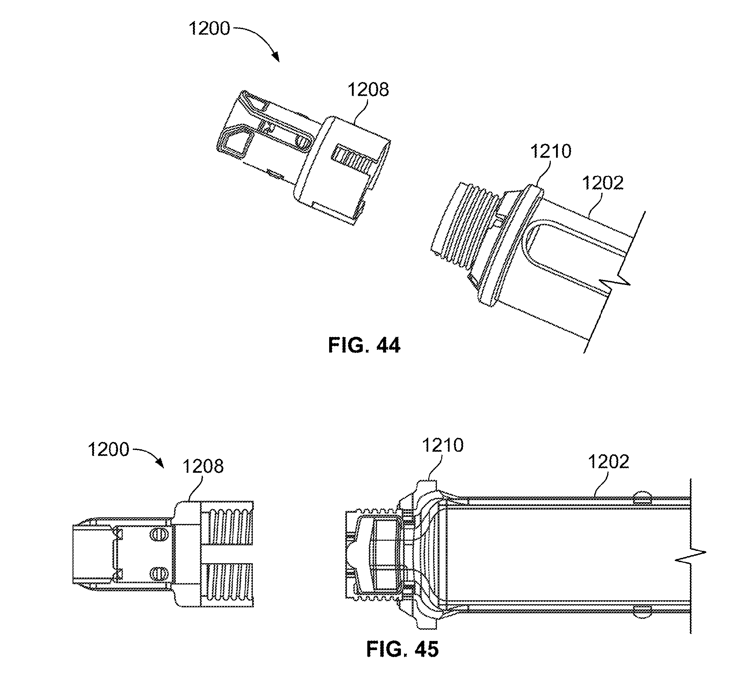

The main body 1206 of the dedicated connector includes a main-body portion 1208 and a neck portion 1210. At least one engagement feature, such as engagement features 1212a-b may be disposed on an inner wall 1214 of the neck portion 1210. The main-body portion 1208 and the neck portion 1210 may be connected by at least one frangible element 1216 (see also Figure 43). The frangible element may be formed in various ways. For instance, as shown in Figure 43, the frangible element 1216 is formed by perforations between the main-body portion 1208 and the neck portion 1210. When the dedicated connector 1200 is removed from the cartridge holder 1202 after the at least one engagement feature is engaged to the corresponding engagement feature, the at least one frangible element 1216 breaks, thereby causing the neck portion 1210 to remain attached to the cartridge holder 1202. Figures 44 and 45 depict when the connector is removed, but the neck portion 1210 remains disposed on the cartridge holder 1202.

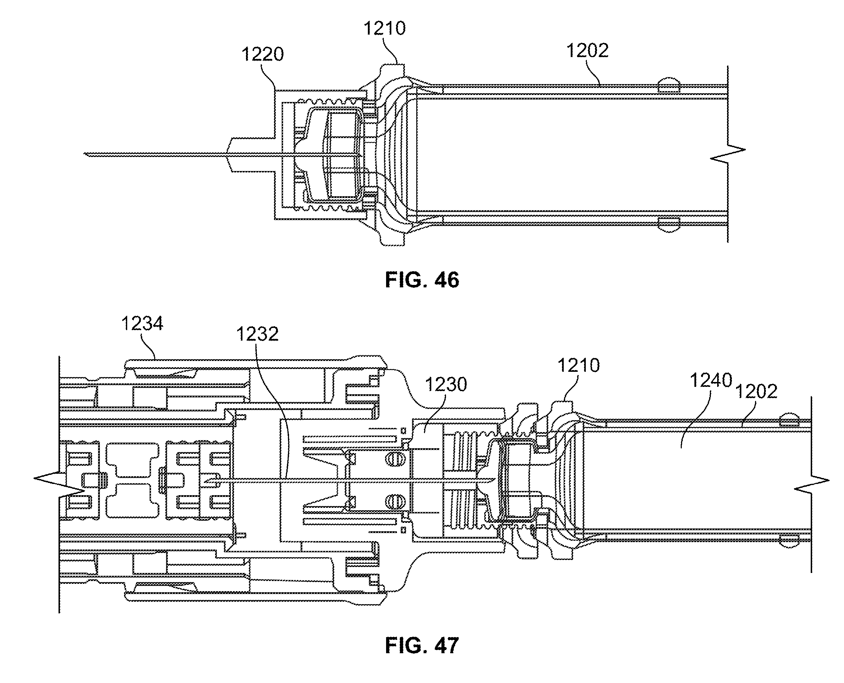

An example benefit of dedicated connector 1200 is that the connector 1200 can be removed so that a conventional needle assembly may be attached to the cartridge holder. This may be useful, for example, to provide a patient or other user with an 'emergency option' for use in instances where, for example, a medicated module (assumed to be only available via prescription) is not available (e.g. the user may have run out, or there is a problem with the one they are trying to use) but the user has access to a standard Type-A needle assembly (widely available, non-prescription). Figure 46 depicts a cross-sectional view of a conventional needle assembly 1220 and the cartridge holder 1202. As can be seen, the neck portion 1210 does not prevent the conventional needle assembly from being screwed on. Thus, a user will be able to use the device with a conventional needle assembly 1220.

However, a new connector will be prevented from being attached to the cartridge holder 1202. As shown in Figure 47, a new connector 1230 may be able to be partially screwed onto the cartridge holder 1202. However, the retained neck portion 1210 will prevent the new connector 1230 from being screwed on enough such that the needle 1232 of the medicated module 1234 will be able to achieve fluid engagement with the cartridge 1240 held in the cartridge holder 1202. Thus, the user will be unable to administer a dose of medicament with the drug delivery device.

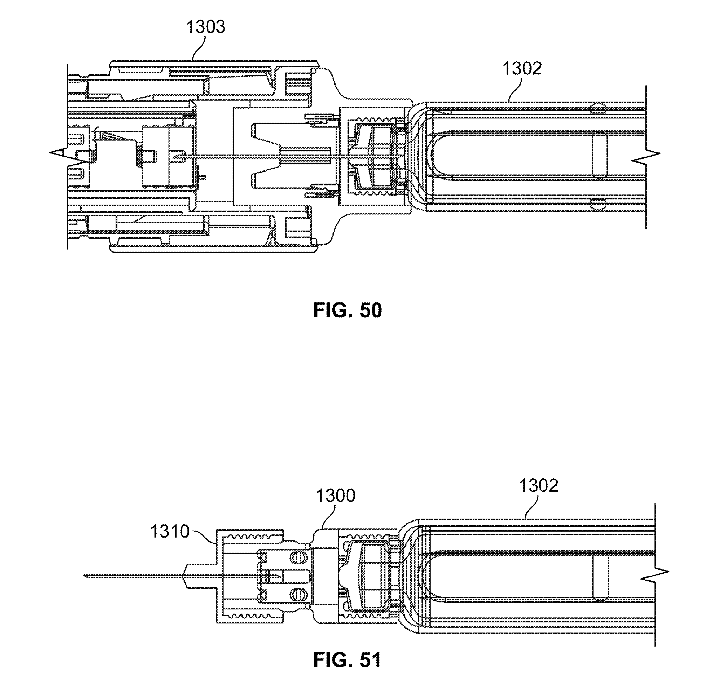

Another example dedicated connector is shown in Figure 48. This example dedicated connector is similar to the dedicated connector 851; however, the dedicated connector of Figure 48 is not permanently connected to the cartridge holder. Rather, the dedicated connector 1300 is releasably connected to dedicated cartridge holder 1302— that is, the dedicated connector can be removed from (e.g., screwed on and screwed off) the cartridge holder. Figure 49 depicts a cross-sectional view of the dedicated connector 1300 connected to the cartridge holder 1302 and the needle assembly 1303. When a cartridge holder does not have the connector 1300, the dedicated needle assembly 1303 will be unable to be properly attached to the cartridge holder 1302. For instance, as seen in Figure 50, the needle of the needle assembly 1303 may be able to enter fluidic engagement with the cartridge, but lack of substantial mechanical engagement will hamper mechanical retention on the cartridge holder. As such, it may be obvious to a user that the needle assembly 1303 is not well retained, and thus the lack of retention may serve as an indication for the user that the device should not be used at that time.

As with the other dedicated connectors discussed above, the dedicated connector 1300 is configured to prevent a needle of a conventional needle assembly from piercing a cartridge septum of a cartridge disposed in the cartridge holder. Figure 51 depicts a conventional needle 1310 attempted to be attached to the connector 1300. As can be seen, there is no fluidic or mechanical engagement, and thus the needle assembly 1310 would simply fall off.

In an example of Applicants' proposed concept, if a medicated module were to be used for scenarios where multiple secondary medicaments could be used (e.g. Lantus plus AVE0010, Lantus plus 'Drug B', Lantus plus 'Drug C', etc.), or where multiple (but independently exclusive) combination therapies where to put on market ('Drug A' plus 'Drug X', 'Drug B' plus 'Drug Y', 'Drug C' plus 'Drug Z', etc.) then it may be beneficial for the exclusive attachment connector to be provided with specific coding features /attributes, or as a series of drug-combination specific configurations. This would potentially enable the same basic attachment method to be used (e.g. push to fit, pull to detach) across all products. This would thereby preserve the usability benefits of the selected approach, while also providing a means for mechanical differentiation /dedication to reduce the risk of patient mix up between individual drugs from the family of combination therapies that used the medicated module system.

Exemplary embodiments of the present invention have been described. Those skilled in the art will understand, however, that changes and modifications may be made to these embodiments without departing from the true scope and spirit of the present invention, which is defined by the claims.

### List of references:

- 1: drug delivery device
- 2: septum
- 3: cap
- 4: coupling mechanism
- 5: cartidge holder
- 6: dose setting mechanism
- 8: dose setter
- 9: spindle
- 10: medicated module
- 12: drug delivery device
- 14: cartridge
- 16: medicament
- 22: inner surface
- 24: connecting body
- 26: proximal end
- 28: distal end
- 30: screw thread
- 32: first recess
- 33: exterior surface
- 34: second recess
- 36: reservoir
- 38: second medicament
- 40: injection needle
- 42: first piercing end
- 44: second piercing end
- 46: capsule
- 48: membrane
- 50: membrane
- 52: outer body
- 54: distal end
- 56: proximal end
- 60: male member
- 61: firs inner cavity
- 62: second inner cavity
- 63: inner surface
- 65: distal groove
- 66: proximal groove
- 68: movable locking member
- 70: elastic member
- 80: injection needle
- 82: first piercing end
- 90: needle guard
- 96: outwardly directed arms
- 98: outwardly directed arms
- 110: proximal direction
- 120: distal direction
- 210: medicated module
- 212: drug delivery device
- 213: threads
- 214: cartridge
- 218: membrane
- 224: connecting body
- 226: proximal end
- 228: distal end
- 230: connector
- 231: main stem
- 235: needle hub
- 239: recess
- 261: first inner cavity
- 268: movable locking member
- 270: biasing member
- 272: spring fingers
- 274: ledge
- 280: distal end needle
- 282: first piercing end
- 284: second piercing end
- 290: needle guard
- 296/298: outwardly directed arms
- 300: needle assembly
- 310: dedicated mechanical coupling
- 320: outer cavity body
- 322: cylindrical shape
- 326: outer surface
- 330: distal end
- 336: proximal end
- 340: outer surface
- 350: proximal end surface
- 360: cavity
- 361: cylindrical shaped opening
- 364: first engaging cavity
- 368: second engaging cavity
- 372: inner wall
- 376: first tongue
- 380: second tongue
- 384: recess feature
- 386: recess feature
- 400: dedicated delivery device
- 404: distal end
- 408: distal portion
- 410: dedicated cartridge housing
- 411: cartridge
- 414: dedicated mechanical coupling
- 415: medicament
- 426: first body portion
- 427: outer surface
- 434: first groove
- 435: first width
- 436: second body portion
- 440: second groove
- 441: second width
- 444: bump feature
- 450: bump feature
- 460: upstand feature
- 462: lip
- 468: upstand feature
- 470: lip
- 486: outer diameter
- 490/497: first chamfer edge
- 496/498: second chamfer edge
- 500: dedicated cap
- 502: main body
- 504: distal end
- 514: dedicated mechanical coupling
- 516: cylindrical extension
- 518: inner diameter
- 524: proximal end
- 526: first body portion
- 527: smooth outer surface
- 530: distal end
- 534/540: grooves
- 535: first width
- 536: second body portion
- 541: second width
- 544/550: bump features
- 560/568: upstand features
- 586: outer diameter
- 590: chamfer edge
- 596: chamfer edge
- 600: needle assembly
- 601: hub
- 602: protrusion
- 603: sleeve
- 604: thread
- 606: double ended needle
- 650: needle assembly
- 652: dedicated mechanical coupling
- 654: connecting body
- 658: distal end
- 660: proximal end
- 663: bump feature
- 665: bump feature
- 668: ring shaped extension
- 672: sl it
- 674: slit
- 676: sl it
- 686: ring shaped cavity
- 688: rib
- 689: corresponding recess
- 690: rib
- 691: corresponding recess
- 692: rib
- 700: needle assembly
- 702: dedicated mechanical coupling
- 718: ring shaped extension
- 719: inner edge
- 721: non-circular outer edge
- 730: drug delivery device
- 736: ring shaped cavity
- 800: needle assembly
- 817: inner edge
- 818: ring shaped extension
- 802: dedicated mechanical coupling
- 822: indentation
- 824: indentation
- 830: drug delivery device
- 836: ring shaped extension
- 837: outer edge
- 838: corresponding protrusion
- 840: corresponding protrusion
- 842: corresponding protrusion
- 851: dedicated connector
- 853: drug delivery device
- 854: cartridge holder
- 855: dedicated needle assembly
- 857: main body
- 859: distal end
- 861: proximal end
- 863: distal end
- 865: dedicated mechanical coupling
- 867: proximal end
- 869: conventional cartridge holder mount
- 871: proximal needle
- 875: needle shroud
- 880: bonding surfaces
- 881: threads
- 882: bonding surfaces
- 883: threads
- 900: dedicated connector
- 902: cartridge holder
- 903: dedicated medicated module
- 904: arm feature
- 906: arm feature
- 908: clip feature
- 910: clip feature
- 912: clip feature
- 914: clip feature
- 916: threads
- 918: bonding surface
- 920: bonding surface
- 922: bonding surface
- 924: bonding surface
- 926: threads
- 928: bonding surface
- 930: neck
- 932: bonding surface
- 934: other side
- 940: needle
- 942: septum
- 944: cartridge
- 946: conventional needle assembly
- 948: conventional needle
- 1000: dedicated connector
- 1002: modified cartridge holder
- 1003: dedicated medicated module
- 1004a-d: four engagement features
- 1006a-b: corresponding engagement features
- 1010: flexible wall
- 1100: dedicated connector
- 1102: modified cartridge holder
- 1103: dedicated medicated module
- 1105: internal thread
- 1106a-b: engagement features
- 1108a-b: corresponding engagement features
- 1200: dedicated connector
- 1202: modified cartridge holder
- 1203: dedicated medicated module
- 1206: main body
- 1208: main-body portion
- 1210: neck portion
- 1212a-b: engagement features
- 1214: inner wall
- 1216: frangible element
- 1220: conventional needle assembly
- 1230: new connector
- 1232: needle
- 1234: medicated module
- 1240: cartridge
- 1300: dedicated connector
- 1302: dedicated cartridge holder
- 1303: needle assembly
- 1310: conventional needle

## Claims

1. A dedicated connector (851, 900, 1000, 1100, 1200, 1300) comprising:
a main body (857) extending from a distal end (859) to a proximal end (861), wherein the proximal end (861) of the main body is configured for attaching to a distal end (863) of a drug delivery device (853, 902, 1002, 1102, 1202, 1302);
a dedicated mechanical coupling (865) configured at the distal end (859) of the main body, wherein the dedicated mechanical coupling (865) is configured to form a connection to a proximal end (867) of a dedicated needle assembly (855, 903, 1003, 1103, 1203, 1303).

2. The dedicated connector of claim 1, wherein the connection is a releasable connection.

3. The dedicated connector of claims 1-2, wherein the proximal end (863) of the main body (857) is permanently fixed to the distal end (863) of the drug delivery device (853, 902, 1002, 1102, 1202, 1302).

4. The dedicated connector of claim 3, wherein the proximal end (863) of the main body (857) is bonded to the distal end (863) of the drug delivery device (853, 902, 1002, 1102, 1202, 1302).

5. The dedicated connector of claims 1-2, wherein the proximal end (863) of the main body (857) is removably fixed to the distal end (863) of the drug delivery device (853, 902, 1002, 1102, 1202, 1302).

6. The dedicated connector of any of the preceding claims, wherein said dedicated mechanical coupling (865) is configured to prevent a connection of said dedicated connector (851, 900, 1000, 1100, 1200, 1300) to a standard needle assembly (946, 1310), wherein the standard needle assembly (946, 1310) comprises a conventional mechanical coupling.

7. The dedicated connector of any of the preceding claims, wherein the dedicated mechanical coupling (865) is configured such that the dedicated needle assembly (855, 903, 1003, 1103, 1203, 1303) is at least one of (i) pushed on the dedicated connector (851, 900, 1000, 1100, 1200, 1300) when the dedicated needle assembly is attached to the drug delivery device (853, 902, 1002, 1102, 1202, 1302), (ii) twisted onto the dedicated connector (851, 900, 1000, 1100, 1200, 1300) when the dedicated needle assembly (855, 903, 1003, 1103, 1203, 1303) is attached to the drug delivery device (853, 902, 1002, 1102, 1202, 1302), and (iii) latched onto the dedicated connector (851, 900, 1000, 1100, 1200, 1300) when the dedicated needle assembly (855, 903, 1003, 1103, 1203, 1303) is attached to the drug delivery device (853, 902, 1002, 1102, 1202, 1302).

8. The dedicated connector of any of the preceding claims, wherein the dedicated mechanical coupling (865) is configured such that the dedicated needle assembly (855, 903, 1003, 1103, 1203, 1303) is at least one of (i) pulled off the dedicated connector (851, 900, 1000, 1100, 1200, 1300) when the dedicated needle assembly (855, 903, 1003, 1103, 1203, 1303) is detached from the dedicated drug delivery device (853, 902, 1002, 1102, 1202, 1302), (ii) twisted off the dedicated connector (851, 900, 1000, 1100, 1200, 1300) when the dedicated needle assembly (855, 903, 1003, 1103, 1203, 1303) is detached from the dedicated drug delivery device (853, 902, 1002, 1102, 1202, 1302), and (iii) unlatched from the dedicated connector (851, 900, 1000, 1100, 1200, 1300) when the dedicated needle assembly (855, 903, 1003, 1103, 1203, 1303) is detached from the dedicated drug delivery device (853, 902, 1002, 1102, 1202, 1302).

9. The dedicated connector of any of the preceding claims, wherein the distal end of a drug delivery device comprises a cartridge holder (854, 902, 1302), wherein the cartridge holder (854, 902, 1302) comprises a conventional mechanical coupling.

10. The dedicated connector of any of the preceding claims, further comprising at least one arm feature (904, 906) protruding from the proximal end (861) of the main body (857), wherein the at least one arm feature (904, 906) is adapted to be at least one of:
i) adapted to be bonded to the drug delivery device (902),
ii) comprises at least one clip feature (908, 910), wherein each of the at least one clip feature (908, 910) is configured to interact with a corresponding clip feature (912, 914) on the drug delivery device (902).

11. The dedicated connector of any preceding claim 1-9, further comprising at least one engagement feature (1004a-d), wherein each of the at least one engagement feature (1004a-d) is configured for interacting with a respective corresponding engagement (1006a-b) feature disposed on the drug delivery device (1002).

12. The dedicated connector of claim 11, wherein the proximal end (861) of the main body (857) comprises a flexible wall (1010), wherein the the at least one engagement feature (1004a-d) comprises a snap engagement feature (1004a-d) disposed on an inner edge of the flexible wall (1010), and wherein the flexible wall (1010) is configured to deflect outward so that the each of the at least one snap engagement feature (1004a-d) can deflect over and engage with the corresponding engagement feature (1006a-b) when the dedicated connector (1000) is connected to the drug delivery device (1002).

13. The dedicated connector of claim 11, wherein the proximal end (861) of the main body (857) further comprises a thread (1105), wherein the at least one engagement feature (1106a-b) prevents the dedicated connector (1100) from being disengaged from the drug delivery device (1102) after each of the at least one engagement feature (1106a-b) engages with the respective corresponding engagement feature (1108a-b).

14. The dedicated connector of claim 11, wherein the main body (1206) comprises a main-body portion (1208) and a neck portion (1210), wherein the at least one engagement feature (1212a-b) is disposed on an inner wall of the neck portion (1210), wherein the main-body portion (1208) and the neck portion (1210) are connected by at least one frangible element (1216),
wherein, when the dedicated connector (1200) is removed from the drug delivery device (1202) after the at least one engagement feature (1212a-b) is engaged to the corresponding engagement feature, the at least one frangible element breaks (1216), thereby causing the neck portion (1210) to remain attached to the drug delivery device (1202).

15. The dedicated connector of claims 1-9, wherein the proximal end (861) of the main body (857) is configured to screw on a cartridge holder (1302) having a conventional mechanical coupling and wherein the dedicated connector (1300) is configured to prevent a needle of a conventional needle assembly (1310) from piercing a cartridge septum of a cartridge disposed in the drug delivery device (1302).
